(19) Europäisches Patentamt
European Patent Office
Office européen des brevets

(11) EP 2 642 581 B1

(12) **EUROPEAN PATENT SPECIFICATION**

(45) Date of publication and mention of the grant of the patent:
**12.04.2017 Bulletin 2017/15**

(21) Application number: **11841961.3**

(22) Date of filing: **15.11.2011**

(51) Int Cl.:
***C07C 22/00*** *(2006.01)* ***H01G 11/60*** *(2013.01)*
***H01M 10/052*** *(2010.01)* ***H01M 10/0569*** *(2010.01)*

(86) International application number:
**PCT/JP2011/006365**

(87) International publication number:
**WO 2012/066770 (24.05.2012 Gazette 2012/21)**

(54) **USE OF A NON-AQUEOUS SOLVENT FOR ELECTRICITY STORAGE DEVICE, AND NON-AQUEOUS ELECTROLYTIC SOLUTION COMPRISING SAID NON-AQUEOUS SOLVENT**

VERWENDUNG EINES NICHTWÄSSRIGEN LÖSUNGSMITTELS FÜR STROMSPEICHERVORRICHTUNG, UND NICHTWÄSSRIGE ELEKTROLYTLÖSUNG DIESES LÖSUNGSMITTEL UMFASSEND

UTILISATION D'UN SOLVANT NON-AQUEUX POUR DISPOSITIF DE STOCKAGE D'ÉLECTRICITÉ, ET SOLUTION ÉLECTROLYTIQUE NON-AQUEUSE COMPRENANT CE SOLVANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2010 US 414151 P**
**26.05.2011 US 201161490423 P**

(43) Date of publication of application:
**25.09.2013 Bulletin 2013/39**

(73) Proprietor: **Panasonic Intellectual Property Management Co., Ltd.**
**Osaka-shi, Osaka 540-6207 (JP)**

(72) Inventors:
- **TAKEUCHI, Takashi**
  **Osaka 540-6207 (JP)**
- **HASEGAWA, Masaki**
  **Osaka 540-6207 (JP)**
- **GOTO, Natsumi**
  **Osaka 540-6207 (JP)**

(74) Representative: **TBK**
**Bavariaring 4-6**
**80336 München (DE)**

(56) References cited:
**EP-A1- 1 557 404** **EP-A1- 1 560 236**
**WO-A1-2009/141999** **JP-A- 2000 223 151**
**JP-A- 2000 348 762** **JP-A- 2001 143 749**
**JP-A- 2006 092 815** **JP-A- 2006 172 775**

- **M.B. EWING ET AL: "An ebulliometer for measurements of vapour pressure at low temperatures: the vapour pressures and the critical state of perfluoromethylcyclopentane", THE JOURNAL OF CHEMICAL THERMODYNAMICS, vol. 30, no. 2, 1 February 1998 (1998-02-01), pages 189-198, XP055213672, ISSN: 0021-9614, DOI: 10.1006/jcht.1997.0290**
- **W. DMOWSKI ET AL: "Selective reactions of 1,1-cycloalkanedicarboxylic acids with SF4. A route to 1,1-bis(trifluoromethyl)cycloalkanes, 1-fluoroformyl-1-(trifluoromethyl)cycloalk anes and 1-(trifluoromethyl)-1-cycloalkanecarboxyli c acids", JOURNAL OF FLUORINE CHEMISTRY, vol. 102, no. 1-2, 1 March 2000 (2000-03-01), pages 141-146, XP004193140, ISSN: 0022-1139, DOI: 10.1016/S0022-1139(99)00233-X**
- **C. MELERO ET AL: "New Modes of Reactivity in the Threshold of the Reduction Potential in Solution. Alkylation of Lithium PAH (Polycyclic Aromatic Hydrocarbon) Dianions by Primary Fluoroalkanes: A Reaction Pathway Complementing the Classical Birch Reductive Alkylation", CHEMISTRY - A EUROPEAN JOURNAL, vol. 13, no. 36, 6 November 2007 (2007-11-06), pages 10096-10107, XP055213832, WEINHEIM, DE ISSN: 0947-6539, DOI: 10.1002/chem.200700187**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 642 581 B1

- **T.B. PATRICK ET AL: "Mechanistic studies of fluorodecarboxylation with xenon difluoride", THE JOURNAL OF ORGANIC CHEMISTRY, vol. 58, no. 3, 1 January 1993 (1993-01-01), pages 705-708, XP055213652, ISSN: 0022-3263, DOI: 10.1021/jo00055a026**
- **J. GONZALEZ ET AL: "Difluoromethylation of alkenes via borohydride reduction of 1,3-dibromo-1,1-difluoroalkanes", THE JOURNAL OF ORGANIC CHEMISTRY, vol. 56, no. 13, 1 June 1991 (1991-06-01), pages 4322-4325, XP055213663, ISSN: 0022-3263, DOI: 10.1021/jo00013a051**

**Description**

## TECHNICAL FIELD

The present invention relates to a nonaqueous solvent to be used in an electrical storage device for stockpiling or storing electrochemical energy.

## BACKGROUND ART

The development of such a high-voltage type electrical storage device, in which the charging voltage and discharge voltage of the electrical storage element, each exceeding 1.5 V, has been advanced in recent years, and a lithium primary battery, a lithium ion secondary battery, a lithium polymer secondary battery, an electric double layer capacitor, or the like has been put into practical use as such high-voltage type electrical storage device.

A nonaqueous electrolytic solution using an organic compound as a solvent is used in the high-voltage type electrical storage device. This is because the use of water as the solvent of the electrolytic solution causes the electrolysis of water due to high charging voltage and high discharge voltage. The nonaqueous electrolytic solution is also used in an electrical storage device, which contains active lithium that reacts with water, and is equipped with an electrode utilizing the occlusion or release of lithium.

It is desired that the nonaqueous electrolytic solution have high conductivity and a low viscosity in order that the discharge performance of an electrical storage device, in which the electrolytic solution is to be used, may be improved. In addition, when the electrolytic solution is used as a solvent for a secondary battery, an electric double layer capacitor, or the like, the electrolytic solution is required to be chemically and electrochemically stable so that the performance of the electrical storage device does not deteriorate due to repeated charge and discharge.

From those viewpoints, a mixed system of a cyclic carbonate typified by ethylene carbonate and a chain carbonate typified by ethyl methyl carbonate or dimethyl carbonate has conventionally been used as, for example, the main solvent of an electrolytic solution for a lithium ion secondary battery. In addition, a cyclic carbonate typified by propylene carbonate has been used as the main solvent of an electrolytic solution for an electric double layer capacitor.

Such electrical storage devices as described above have been widely utilized as main power sources for mobile communication equipment and portable electronic equipment, backup power sources, and power sources for electric circuits. Each of the equipment has been required to be smaller in size and to have higher performance in recent years, and hence further enhancement in the volume energy density of any of the electrical storage devices has been requested.

The enhancement of the volume energy density requires realization of an increase in average discharge voltage and the enhancement of the volume capacity density, and investigations have been conducted on an increase in charging voltage as one means for the realization.

In the case of a lithium ion secondary battery, increasing its charging voltage can improve the efficiency with which lithium as a positive electrode material is utilized, thereby increasing its volume capacity density. A lithium-containing, laminar transition metal oxide such as lithium cobaltate or lithium nickelate is generally used as the positive electrode material. In addition, in the case of an electric double layer capacitor, increasing its charging voltage can increase a value for its electric double layer capacity and hence can increase its volume capacity density.

However, in the case where one electrode of a pair of electrodes is charged to 4.3 V or more with reference to the dissolution-precipitation potential of lithium, even when conventional chain carbonates and cyclic carbonates known as nonaqueous solvents excellent in oxidation resistance and suitable for high-voltage type electrical storage devices are used, the oxidative decomposition of any such carbonate occurs to generate a gas. The decomposition reaction progresses in a remarkable fashion particularly in a high-temperature state, and involves the generation of a large amount of the gas. Accordingly, when a high-voltage type lithium ion secondary battery containing any such nonaqueous solvent is mounted with, for example, an internal pressure-sensitive current interrupt device that interrupts a charging current in response to the overcharge of the battery, the CID may operate in a wrong fashion to impair a function of the battery. In addition, when no CID is mounted, there may arise a problem in that an increase in the generation amount of the gas causes the expansion of the battery.

Patent Document No. 1 discloses a nonaqueous electrolyte secondary battery using a nonaqueous electrolytic solution containing a cyclic sulfonate for suppressing the oxidative decomposition of chain carbonates and cyclic carbonates under an ultrahigh potential. In such nonaqueous electrolyte secondary battery, when its positive electrode is charged to a potential of 4.5V or more, the cyclic sulfonate undergoes oxidative decomposition on a positive electrode side, and hence a coating is formed on the surface of the positive electrode. The formation of the coating suppresses the decomposition of a solvent on the surface of the positive electrode.

Meanwhile, Patent Document No. 2 and Patent Document No. 3 each propose the incorporation of 0.01 wt% or more and 5 wt% or less of a "hydrocarbon compound which may have a fluorine atom" into a nonaqueous solvent. Those patent literatures each describe that the presence of the hydrocarbon compound stable toward oxidation and

reduction at an active site on the surface of an electrode can suppress a side reaction between an electrolytic solution component and an electrode active material in a high-temperature state.

Further, Patent Document No. 4 proposes the use of a fluorine-containing, cyclic, saturated hydrocarbon obtained by introducing one or two fluorine-substituted alkyl groups into a cyclohexane ring as a solvent. The patent literature describes that the fluorine-containing, cyclic, saturated hydrocarbon has high oxidation resistance and can suppress the generation of a gas involved in the oxidative decomposition of the solvent even when a positive electrode is brought into an ultrahigh voltage state of 4.4 V with respect to the dissolution-precipitation potential of lithium.

EP 1 557 404 A1; M.B. EWING et al., THE JOURNAL OF CHEMICAL THERMODYNAMICS, (19980201), vol. 30, no. 2, pp. 189 - 198; W. DMOWSKI et al., JOURNAL OF FLUORINE CHEMISTRY, (20000301), vol. 102, no. 1-2, pp. 141 - 146; C. MELERO et al., CHEMISTRY - A EUROPEAN JOURNAL, (20071106), vol. 13, no. 36, pp. 10096 - 10107; T.B. PATRICK et al., THE JOURNAL OF ORGANIC CHEMISTRY, (19930101), vol. 58, no. 3, pp. 705 - 708; and J. GONZALEZ et al., THE JOURNAL OF ORGANIC CHEMISTRY, (19910601), vol. 56, no. 13, pp. 4322 - 4325, all describe cyclopentane compounds bearing one or two fluoroalkyl substituents.

EP 1 560 236 A1, JP 2001 143749 A, JP 2000 348762 A, JP 2000 223151 A, JP 2006 172775 A and JP 2006 092815 A, all describe non-aqueous electrolytic solutions for electrochemical devices comprising a cyclopentane compound or a fluorine or alkyl substituted derivative thereof as an additive or co-solvent.

## CITATION LIST

### PATENT LITERATURE

Patent Document No. 1: Japanese Patent Application Laid-open No. 2005-149750
Patent Document No. 2: Japanese Patent Application Laid-open No. 2004-111359
Patent Document No. 3: Japanese Patent Application Laid-open No. 2006-286650
Patent Document No. 4: International Publication WO 2009/141999 corresponding to US 2009/0297954.

## SUMMARY OF INVENTION

### TECHNICAL PROBLEM

Although the nonaqueous electrolyte secondary battery disclosed in Patent Document No. 1 can suppress the decomposition reactions of the chain carbonates and the cyclic carbonates, the suppressing effect is not sufficient. Further, the coating is formed on the surface of the positive electrode, with the result that there may arise a problem in that a charge transfer resistance at a positive electrode active material interface increases, and hence the internal resistance of the battery increases and its high-rate discharge performance reduces.

In addition, Patent Document No. 2 and Patent Document No. 3 each describe that the nonaqueous electrolyte secondary battery disclosed therein can suppress the side reaction between the electrolytic solution component and the electrode active material in a high-temperature state with the "hydrocarbon compound which may have a fluorine atom." However, the content of the hydrocarbon compound is as small as 5 wt% or less. In addition, the hydrocarbon compound does not selectively exist at a high concentration on the surface of the positive electrode because the compound does not have such property as to, for example, adsorb or coordinate to the surface of the positive electrode. Therefore, it cannot be said that Patent Document No. 2 and Patent Document No. 3 each exert a sufficient suppressing effect on the side reaction.

Further, the solvent for an electrical storage device disclosed in Patent Document No. 4 is acknowledged to exert an effect from the viewpoint of the suppression of the generation of a gas involved in the oxidative decomposition of the solvent because the solvent has high oxidation resistance. However, the solvent still has room for improvement from the viewpoint of a viscosity as an important parameter for the solvent to play a role of transporting a lithium ion in an electrolytic solution.

An object of the present invention is to provide a nonaqueous solvent for an electrical storage device that has solved at least one of such prior art problems, is excellent in oxidation resistance, generates a small amount of a gas even when decomposed, and has a low viscosity.

### SOLUTION TO PROBLEM

In a first embodiment, the present invention relates to the use of a nonaqueous solvent comprising a specific fluorinated cycloalkane compound for an electrical storage device.

In a second embodiment of the present invention, a nonaqueous electrolytic solution for electrical storage device is disclosed, which solution comprises a supporting electrolyte, at least one of a cyclic carbonate and a chain carbonate, and a fluorinated cycloalkane compound as defined herein below.

The nonaqueous solvent used for an electrical storage device according to an exemplary embodiment of the present invention includes a fluorine-containing, cyclic, saturated hydrocarbon represented by the following general formula (1) and having a structure obtained by introducing one or two substituents R into a cyclopentane ring:

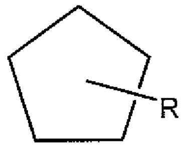 ··· (1)

in the general formula (1), R is represented by $C_nX_{2n+1}$, n represents 1 or 2, at least one of the 2n+1 X's represents F, and the other X's each represent H.

In a preferred embodiment, the n represents 1 or 2.

In a preferred embodiment, the fluorine-containing, cyclic, saturated hydrocarbon includes trifluoromethylcyclopentane.

In a preferred embodiment, the fluorine-containing, cyclic, saturated hydrocarbon includes 1,1-bis(trifluoromethyl)cyclopentane.

In a preferred embodiment, the fluorine-containing, cyclic, saturated hydrocarbon includes 2-fluoroethylcyclopentane.

In a preferred embodiment, the nonaqueous solvent for an electrical storage device contains 5 wt% or more and 100 wt% or less of the fluorine-containing, cyclic, saturated hydrocarbon as a solvent component.

In a preferred embodiment, the nonaqueous solvent for an electrical storage device contains 10 wt% or more and 100 wt% or less of the fluorine-containing, cyclic, saturated hydrocarbon as a solvent component.

## ADVANTAGEOUS EFFECTS OF INVENTION

The nonaqueous solvent used for an electrical storage device according to the exemplary embodiment of the present invention has high oxidation resistance and compatibility with an organic solvent to be generally used in an electrical storage device because the solvent contains the fluorine-containing, cyclic, saturated hydrocarbon having a cyclopentane skeleton with a substituent into which fluorine is introduced. The fluorine-containing, cyclic, saturated hydrocarbon hardly produces a gas owing to its oxidative decomposition because the hydrocarbon is free of oxygen. In addition, the hydrocarbon has a small viscosity and excellent diffusion property because of its small molecular volume.

## BRIEF DESCRIPTION OF DRAWINGS

[FIG. 1] A view illustrating a relationship between the dipole moment and highest occupied molecular orbital (HOMO) of each fluorine-containing, cyclic, saturated hydrocarbon for constructing a nonaqueous solvent for an electrical storage device according to an exemplary embodiment of the present invention.

[FIG. 2] A view illustrating the molecular volumes of each fluorine-containing, cyclic, saturated hydrocarbon for constructing the nonaqueous solvent for an electrical storage device according to the exemplary embodiment of the present invention, and a fluorine-containing, cyclic, saturated hydrocarbon having a corresponding substituent and containing a cyclohexane skeleton.

[FIG. 3] A sectional view illustrating a triode glass cell used in an evaluation for oxidation resistance.

[FIG. 4] A graph of a voltage-current curve illustrating an oxidation resistance of Example 4.

[FIG. 5] (a) is a perspective view of a laminate type lithium ion secondary battery used for producing a charged positive electrode in the experiment of Example 5, (b) is a sectional view along the line I-I of FIG. 5(a), and (c) is an enlarged view illustrating a section of an electrode group 13 illustrated in each of FIG. 5(a) and FIG. 5(b).

[FIG. 6] A flowchart illustrating a procedure for the experiment of Example 5.

[FIG. 7] (a) is a view illustrating the size of the positive electrode in Example 5, and (b) is a view illustrating the size of a negative electrode in Example 5.

**DESCRIPTION OF EMBODIMENTS**

Hereinafter, an embodiment of a nonaqueous solvent used for an electrical storage device according to the present invention is described. The nonaqueous solvent of this embodiment is used in an electrical storage device such as a lithium ion secondary battery or an electric double layer capacitor.

The nonaqueous solvent used for an electrical storage device of the present invention contains a fluorine-containing, cyclic, saturated hydrocarbon represented by the following general formula (1). The fluorine-containing, cyclic, saturated hydrocarbon has a structure obtained by introducing one or two substituents R into a cyclopentane ring.

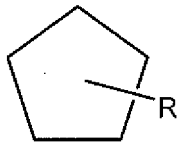 ··· (1)

The substituent R is represented by $C_nX_{2n+1}$. n represents 1 or 2, at least one of the 2n+1 X's represents F, and the other X's each represent H. In other words, the substituent R is a chain, saturated hydrocarbon group at least one hydrogen (H) of which is substituted with fluorine (F).

The fluorine-containing, cyclic, saturated hydrocarbon represented by the general formula (1) has a higher oxidation-reduction potential than those of a cyclic carbonate and a chain carbonate, specifically an oxidation-reduction potential of 4.3 V or more, and hence improves the oxidation resistance of the nonaqueous solvent for an electrical storage device. In addition, the hydrocarbon does not generate $CO_2$ even when decomposed.

In general, a saturated hydrocarbon has high oxidation resistance. However, it has been conventionally considered difficult to use the hydrocarbon as the solvent of a nonaqueous electrolytic solution for an electrical storage device because the hydrocarbon has the following properties. The hydrocarbon has a low dielectric constant and low compatibility with a polar solvent. Accordingly, only such limited usage that an amount as small as 5 wt% or less of the saturated hydrocarbon is incorporated into a solvent has been conventionally proposed like Patent Document No. 2 and Patent Document No. 3.

However, the inventors of the present application have found that as described in detail in the following examples, cyclopentane having, as a substituent, a hydrocarbon group in which hydrogen is substituted with fluorine is excellent in compatibility with a polar solvent because the cyclopentane has low molecular symmetry and a dipole moment of 1. 6 debye or more, and the cyclopentane is excellent in oxidation resistance because the cyclopentane has a cyclic, saturated hydrocarbon skeleton.

The fluorine-containing, cyclic, saturated hydrocarbon represented by the general formula (1) is excellent in oxidation stability because the hydrocarbon is free of any functional group poor in oxidation stability in a molecule thereof. In addition, a fluorine atom bonded to the substituent R has a strong electron-withdrawing effect, and hence the oxidation resistance of the cyclic, saturated hydrocarbon can be additionally improved as compared with that in the case where the hydrocarbon is not substituted with fluorine.

The cyclic, saturated hydrocarbon preferably has a cyclic structure that is five-membered or more because of the following reasons. Such hydrocarbon is a liquid in such a temperature range that an electrical storage device is used, and is easily available and easy to handle. The role of a solvent in an electrolytic solution for an electrical storage device is, for example, to quickly perform the transport of a lithium cation when the device is a lithium ion secondary battery, or the transport of a quaternary ammonium cation and a tetrafluoroboric acid anion when the device is an electric double layer capacitor. Therefore, it is preferred that the solvent to be used in the electrolytic solution for an electrical storage device have as low a viscosity as possible and diffuse at as high a rate as possible in the electrolytic solution. The diffusion motion of a molecule in a solution can be represented with the Einstein-Stokes equation represented by the following formula (2).

$$D = \frac{kT}{6\pi r \eta} \quad \cdots \quad (2)$$

In the equation, D represents the diffusion coefficient of a solvent molecule, k represents Boltzmann's constant, T represents an absolute temperature, r represents a molecular radius, and $\eta$ represents the viscosity of a microscopic ambient environment where the solvent molecule is placed. It is understood from the formula (2) that reducing the molecular radius, in other words, a molecular volume is effective in increasing the diffusion coefficient D of the solvent

molecule. It is understood from the formula (2) that when the same substituent R is introduced, for example, the diffusion coefficient D of a solvent molecule of cyclohexane is smaller than that of cycloheptane, and the diffusion coefficient D of cyclopentane is smaller than that of cyclohexane.

In addition, the compound represented by the general formula (1) in which fluorine is bonded to the substituent R has lower molecular symmetry than that of a compound in which a fluorine atom is directly bonded to a cyclopentane ring such as 1,2-difluorocyclopentane or 1,1,2,2,3,3,4,5-octafluorocyclopentane, and hence the compound represented by the general formula (1) has relatively large polarity and a relatively large dielectric constant. Accordingly, the fluorine-containing, cyclic, saturated hydrocarbon represented by the general formula (1) is excellent in compatibility with a polar solvent.

As the number of fluorine atoms in the substituent R increases, the extent to which the substituent R withdraws electrons from the cyclopentane ring enlarges, and hence the oxidation resistance of the cyclopentane ring is improved. Therefore, the substituent R is more preferably a trifluoromethyl group or a pentafluoroethyl group.

In addition, the number of the substituents R is preferably one or two. When the number of the substituents R is two, the positions at which the substituents R are introduced into cyclopentane are not particularly limited, provided that from the viewpoint of a reduction in its melting point, the compound preferably has such a molecular structure that one substituent R is bonded to the carbon atom adjacent to the carbon atom to which the other substituent R is bonded, and more preferably has such a molecular structure that the two substituents R are bonded to the same carbon atom.

When the number of the substituents R is two, the two substituents R may have structures identical to each other, or may have different structures. When the number of the substituents R is larger than two, the diffusion rate of a molecule reduces because its molecular weight increases. In addition, when the substituent R is excessively large, the diffusion rate of the molecule reduces because its molecular weight increases. Accordingly, the number of carbon atoms (n) of the R is preferably one or two.

Specific examples of the fluorine-containing, cyclic, saturated hydrocarbon compound represented by the general formula (1) include fluoromethylcyclopentane,difluoromethylcyclopentane, trifluoromethylcyclopentane, (1-fluoroethyl)cyclopentane, (2-fluoroethyl)cyclopentane, (1,1-difluoroethyl)cyclopentane, (1,2-difluoroethyl)cyclopentane, (2,2-difluoroethyl)cyclopentane, (1,1,2-trifluoroethyl)cyclopentane, (1,2,2-trifluoroethyl)cyclopentane, (2,2,2-trifluoroethyl)cyclopentane, (1,1,2,2-tetrafluoroethyl)cyclopentane, (1,2,2,2-tetrafluoroethyl)cyclopentane, (pentafluoroethyl)cyclopentane, 1,1-bis(trifluoromethyl)cyclopentane, 1,2-bis(trifluoromethyl)cyclopentane, 1,3-bis(trifluoromethyl)cyclopentane, 1-(pentafluoroethyl)-1-(trifluoromethyl)cyclopentane, 1-(pentafluoroethyl)-2-(trifluoromethyl)cyclopentane, 1-(pentafluoroethyl)-3-(trifluoromethyl)cyclopentane, 1,1-bis(pentafluoroethyl)cyclopentane, 1,2-bis(pentafluoroethyl)cyclopentane, and 1,3-bis(pentafluoroethyl)cyclopentane.

From the viewpoints of oxidation resistance and viscosity, it is particularly preferred to use trifluoromethylcyclopentane, (pentafluoroethyl)cyclopentane, 1,1-bis(trifluoromethyl)cyclopentane as the fluorine-containing, cyclic, saturated hydrocarbon.

Those compounds may be synthesized by a fluorination method using fluorinating reagents such as $F_2$, $NF_3$, HF, $XeF_2$, $SF_4$, $CF_3I$, $C_2F_5I$, (dimethylamino)sulfur trifluoride (DAST), bis(2-methoxyethyl)aminosulfur trifluoride, tetrabutylammonium fluoride, and trimethyl(trifluoromethyl)silane. More specifically, the above-mentioned compounds may be synthesized by, for example, fluorinating an OH group using DAST, fluorinating a COOH group using $SF_4$, and fluorinating C=C using $F_2$.

A starting material is not particularly limited, and a compound having, for example, a cyclopentane skeleton, a cycloheptene skeleton, or a cyclohepta-1,3-diene skeleton can be used. A target compound can be synthesized by introducing an alkyl group to which a hydroxyl group, a tosyl group, a ketone group, or a carboxyl group is bonded into a site to be fluorinated of the starting material, and fluorinating the material with the fluorinating reagent. Alternatively, depending on the structure of the target compound, the compound represented by the formula (1) can be synthesized by directly introducing a hydroxyl group, a tosyl group, a ketone group, or a carboxyl group to the starting material, and fluorinating the material with the fluorinating reagent described above.

The nonaqueous solvent for an electrical storage device of this embodiment has high oxidation resistance and a low viscosity by the reasons described above. In addition, the nonaqueous solvent for an electrical storage device of this embodiment can be added at a high ratio to the nonaqueous electrolytic solution of an electrical storage device because the solvent is excellent in compatibility with an organic solvent to be generally used as the nonaqueous electrolytic solution of the electrical storage device. Therefore, the use of the nonaqueous solvent for an electrical storage device of this embodiment as the nonaqueous electrolytic solution of an electrical storage device or the addition of the nonaqueous solvent for an electrical storage device of this embodiment to the nonaqueous electrolytic solution of the electrical storage device can improve the oxidation resistance of the nonaqueous electrolytic solution and the diffusion property of a cation or anion in the nonaqueous electrolytic solution.

In addition, even the oxidative decomposition of the solvent for an electrical storage device of this embodiment does not involve the generation of $CO_2$. Therefore, an electrical storage device using the nonaqueous solvent for an electrical storage device of this embodiment can avoid such a problem that a safety mechanism (CID) operates or the

battery expands owing to the oxidative decomposition of the solvent.

As the oxidation of a nonaqueous electrolytic solution in an electrical storage device is dominated by a concentration-dependent reaction rate, any such effect is exerted in accordance with the ratio at which the solvent is added to the nonaqueous electrolytic solution. Therefore, the oxidation resistance of the nonaqueous electrolytic solution of an electrical storage device is improved and the generation of a gas is suppressed as long as the solution contains the nonaqueous solvent for an electrical storage device of this embodiment. In order that a remarkable effect of the present invention may be obtained, the fluorine-containing, cyclic hydrocarbon compound represented by the general formula (1) is preferably incorporated at a content of 5 wt% or more and 100 wt% or less into the solvent. As long as the content in the solvent is 5 wt% or more, the oxidation of the nonaqueous electrolytic solution is effectively suppressed and the generation amount of the gas is reduced. As long as the content is 10 wt% or more and 100 wt% or less, those effects are remarkably obtained.

The nonaqueous solvent for an electrical storage device of this embodiment can be suitably used in a device having a high charging voltage (ultrahigh-breakdown voltage type, nonaqueous electrical storage device). In particular, the use of the nonaqueous solvent for an electrical storage device of this embodiment in an electrical storage device such as a lithium ion secondary battery or an electric double layer capacitor suppresses the oxidative degradation of the nonaqueous electrolytic solution in a high-voltage operation, high-temperature storage, and the repetition of a charge and discharge cycle over a long time period.

When the solvent of this embodiment is used as a nonaqueous electrolytic solution for an electrical storage device such as a lithium ion secondary battery or an electric double layer capacitor, the solvent is used as a mixture with a known supporting electrolyte and a carbonate-based solvent. The kind of the supporting electrolyte or the other solvent to be mixed is not particularly limited.

Salts formed of an anion and a cation are generally used as the supporting electrolyte. Examples of the anion species include a halide anion, perchloric acid anion, trifluoromethane sulfonic acid anion, tetrafluoroboric acid anion, hexafluorophosphoric acid anion, trifluoromethanesulfonic acid anion, nonafluoro-1-butanesulfonic acid anion, bis(trifluoromethanesulfonyl)imide anion, and bis(pentafluoroethylsulfonyl)imide anion, and examples of the cation species include a cation of alkali metals such as lithium, sodium, and potassium, a cation of alkaline earth metals such as magnesium, and a cation of quaternary ammoniums typified by tetraethylammonium and 1,3-ethylmethylimidazolium (EMI).

Examples of the salts formed of anion species and cation species as described above include lithium salts such as $LiClO_4$, $LiBF_4$, $LiPF_6$, $LiCF_3SO_3$, and LiN $(CF_3SO_2)_2$, and quaternary ammonium salts such as $(C_2H_5)_4NBF_4$ and EMI-$BF_4$.

Further, examples of the mixed solvents include cyclic carbonates such as ethylene carbonate, propylene carbonate, butylene carbonate, and vinylene carbonate, and chain carbonates such as dimethyl carbonate, diethyl carbonate, ethylmethyl carbonate, and dipropyl carbonate.

[Example 1]

1. Evaluations of fluorine-containing, cyclic, saturated hydrocarbon represented by general formula (1) for its dipole moment, highest occupied molecular orbital (HOMO) energy, and molecular volume

The dipole moment, highest occupied molecular orbital energy, and molecular volume of a fluorine-containing, cyclic, saturated hydrocarbon represented by the general formula (1) were calculated. For comparison, the dipole moments, highest occupied molecular orbital energies, and molecular volumes of methylcyclohexane (MCH) and methylcyclopentane (MCP) each free of fluorine, and a fluorine-containing, cyclic, saturated hydrocarbon having, as its skeleton, cyclohexane as a six-membered ring were also calculated.

The dipole moment is an indicator representing the magnitude of intramolecular polarization, and relates to compatibility with a polar solvent expressed by the present invention. In addition, the highest occupied molecular orbital energy is an indicator representing energy needed for drawing one electron from a molecule, and relates to the oxidation resistance of a solvent. Further, the molecular volume is an indicator representing the size of a solvent molecule. As the molecular volume reduces, a molecular radius also reduces. Accordingly, as represented by the formula (2), the diffusion property of the solvent molecule is improved.

The dipole moment, the highest occupied molecular orbital energy, and the molecular volume were calculated with a quantum chemical calculation approach. Specifically, the calculation was performed with a commercially available first-principles molecular orbital calculation software, and a density functional method (B3LYP) and a 6-31G(d) were used as the calculation approach and a basis function, respectively. It should be noted that the optimization of an energy value was performed by self-consistent field calculation. The molecular volume was estimated from atomic arrangement based on the result of molecular orbital calculation with a space-filling model.

Table 1 shows the results of the calculation. In addition, FIG. 1 illustrates a view having an axis of abscissa

and an axis of ordinate indicating the dipole moment and highest occupied molecular orbital energy of the fluorine-containing, cyclic, saturated hydrocarbon represented by the general formula (1), respectively. In addition, FIG. 2 illustrates the result of comparison between the molecular volumes of the fluorine-containing, cyclic, saturated hydrocarbon represented by the general formula (1) and a fluorine-containing, cyclic, saturated hydrocarbon having cyclohexane as a skeleton for each substituent (R).

Table 1

| | Compound name | Structural formula | Dipole moment [debye] | Highest occupied molecular orbital energy [eV] | Molecular volume [Å³] |
|---|---|---|---|---|---|
| Reference Example | Fluoromethylcyclohexane | 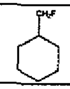 | 1.72 | -8.02 | 135 |
| Reference Example | Difluoromethylcyclohexane | 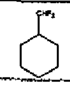 | 2.14 | -8.20 | 140 |
| Reference Example | Trifluoromethylcyclohexane | 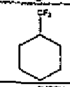 | 2.33 | -8.42 | 144 |
| Reference Example | (1-Fluoroethyl)cyclohexane | 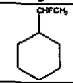 | 1.66 | -7.90 | 153 |
| Reference Example | (2-Fluoroethyl)cyclohexane | 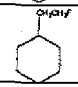 | 1.97 | -7.90 | 154 |
| Reference Example | (1,1-Difluoroethyl)cyclohexane | 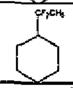 | 1.97 | -8.04 | 158 |
| Reference Example | (1,2-Difluoroethyl)cyclohexane | 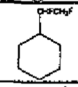 | 2.68 | -8.00 | 158 |
| Reference Example | (2,2-Difluoroethyl)cyclohexane | 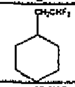 | 2.07 | -7.99 | 158 |
| Reference Example | (1,1,2-Trifluoroethyl)cyclohexane | 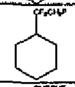 | 3.34 | -8.15 | 163 |
| Reference Example | (1,2,2-Trifluoroethyl)cyclohexane | 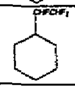 | 2.93 | -8.09 | 163 |

| | Compound name | Structural formula | Dipole moment [debye] | Highest occupied molecular orbital energy [eV] | Molecular volume [Å³] |
|---|---|---|---|---|---|
| Example | Fluoromethylcyclopentane | 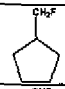 | 1.74 | -8.41 | 119 |
| Example | Difluoromethylcyclopentane | 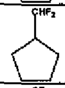 | 2.17 | -8.58 | 123 |
| Example | Trifluoromethylcyclopentane | 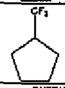 | 2.32 | -8.85 | 128 |
| Example | (1-Fluoroethyl)cyclopentane | 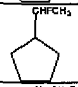 | 1.66 | -8.25 | 137 |
| Example | (2-Fluoroethyl)cyclopentane | 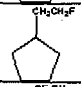 | 1.94 | -8.26 | 137 |
| Example | (1,1-Difluoroethyl)cyclopentane | 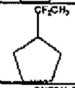 | 1.99 | -8.43 | 142 |
| Example | (1,2-Difluoroethyl)cyclopentane | 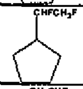 | 2.23 | -8.36 | 142 |
| Example | (2,2-Difluoroethyl)cyclopentane | 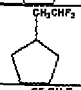 | 2.15 | -8.41 | 142 |
| Example | (1,1,2-Trifluoroethyl)cyclopentane | 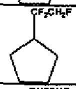 | 3.55 | -8.48 | 146 |
| Example | (1,2,2-Trifluoroethyl)cyclopentane | 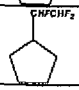 | 3.19 | -8.50 | 146 |

Table 1 Continued

| | Compound name | Structural formula | Dipole moment [debye] | Highest occupied molecular orbital energy [eV] | Molecular volume [Å³] |
|---|---|---|---|---|---|
| Example | (2,2,2-Trifluoroethyl)cyclopentane | 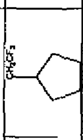 | 2.18 | -8.67 | 146 |
| Example | (1,1,2,2-Tetrafluoroethyl)cyclopentane | 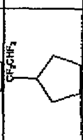 | 2.60 | -8.63 | 151 |
| Example | (1,2,2,2-Tetrafluoroethyl)cyclopentane | 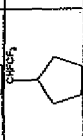 | 2.20 | -8.72 | 151 |
| Example | (Pentafluoroethyl) cyclopentane | 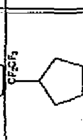 | 2.35 | -8.92 | 155 |
| Example | 1,1-Bis(trifluoromethyl)cyclopentane | 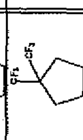 | 2.53 | -9.28 | 159 |
| Example | 1,2-Bis(trifluoromethyl)cyclopentane | 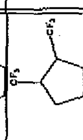 | 3.28 | -9.15 | 159 |
| Example | 1,3-Bis(trifluoromethyl)cyclopentane | 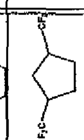 | 1.99 | -9.38 | 160 |
| Example | 1-(Pentafluoroethyl)-1-(trifluoromethyl)cyclopentane | 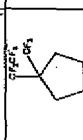 | 2.51 | -9.24 | 186 |
| Example | 1,1-Bis(Pentafluoroethyl)cyclopentane | 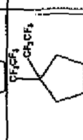 | 2.42 | -9.01 | 213 |
| Comparative Example | Methylcyclopentane | 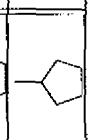 | 0.07 | -8.29 | 114 |

| | Compound name | Structural formula | Dipole moment [debye] | Highest occupied molecular orbital energy [eV] | Molecular volume [Å³] |
|---|---|---|---|---|---|
| Reference Example | (2,2,2-Trifluoroethyl)cyclohexane | 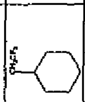 | 2.20 | -8.13 | 163 |
| Reference Example | (1,1,2,2-Tetrafluoroethyl)cyclohexane | 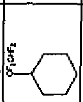 | 2.76 | -8.26 | 167 |
| Reference Example | (1,2,2,2-Tetrafluoroethyl)cyclohexane | 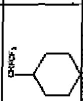 | 2.26 | -8.23 | 167 |
| Reference Example | (Pentafluoroethyl) cyclohexane | 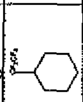 | 2.39 | -8.39 | 172 |
| Reference Example | 1,1-Bis(trifluoromethyl)cyclohexane | 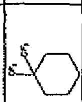 | 2.51 | -8.70 | 175 |
| Reference Example | 1,2-Bis(trifluoromethyl)cyclohexane | 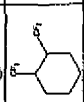 | 3.20 | -8.80 | 176 |
| Reference Example | 1,3-Bis(trifluoromethyl)cyclohexane | 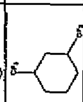 | 2.42 | -8.91 | 176 |
| Reference Example | 1-(Pentafluoroethyl)-1-(trifluoromethyl)cyclohexane | 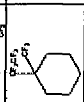 | 2.55 | -8.66 | 203 |
| Reference Example | 1,1-Bis(Pentafluoroethyl)cyclohexane | 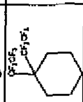 | 2.66 | -8.68 | 230 |
| Comparative Example | Methylcyclohexane | 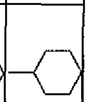 | 0.08 | -7.84 | 130 |

As illustrated in FIG. 1, the dipole moments of the fluorine-containing, cyclic, saturated hydrocarbons represented by the general formula (1) are each about 1.66 debye or more. The fluorine-containing, cyclic, saturated hydrocarbon of the present invention having the smallest dipole moment is (1-fluoroethyl)cyclopentane and its dipole moment is 1.66 debye. In contrast, methylcyclopentane and methylcyclohexane had dipole moments of 0.07 debye and 0.08 debye, respectively.

The examples of Patent Document No. 4 disclose that (1-fluoroethyl)cyclohexane having a dipole moment of 1.66 debye is compatible with propylene carbonate (PC) as a general polar solvent, but methylcyclohexane having a dipole moment of 0.08 debye is not compatible with PC. Therefore, any one of the fluorine-containing, cyclic, saturated hydrocarbons of the present invention is found to have an ability to be compatible with a polar solvent.

In addition, the highest occupied molecular orbital energy of the fluorine-containing, cyclic, saturated hydrocarbon represented by the general formula (1) is comparable to or smaller than that of methylcyclopentane. As an oxidation reaction is a reaction involving drawing an electron from a molecule, it can be said that a compound having smaller (negatively larger) highest occupied molecular orbital energy requires larger energy for drawing an electron and hence has higher oxidation resistance. Accordingly, any one of the fluorine-containing, cyclic, saturated hydrocarbons of the present invention each obtained by introducing an alkyl group having a fluorine atom as a substituent into a cyclopentane ring structure is found to have high oxidation resistance.

In Table 1, each example shown on the left side and each reference example shown on the right side have the same substituent (R). Comparison between the fluorine-containing, cyclic, saturated hydrocarbon represented by the general formula (1) and a fluorine-containing, cyclic, saturated hydrocarbon having cyclohexane as a skeleton for each substituent (R) shows that for any substituent (R), the fluorine-containing, cyclic, saturated hydrocarbon represented by the general formula (1) and having a cyclopentane skeleton has smaller (negatively larger) highest occupied molecular orbital energy than that of the fluorine-containing, cyclic, saturated hydrocarbon having cyclohexane as a skeleton. Therefore, it can be said that the fluorine-containing, cyclic, saturated hydrocarbon represented by the general formula (1) has higher oxidation resistance than that of the fluorine-containing, cyclic, saturated hydrocarbon having, as a skeleton, cyclohexane having the same substituent (R).

Further, as illustrated in FIG. 2, when the fluorine-containing, cyclic, saturated hydrocarbon represented by the general formula (1) and the fluorine-containing, cyclic, saturated hydrocarbon having, as a skeleton, cyclohexane having the same substituent (R) are compared with each other, the fluorine-containing, cyclic, saturated hydrocarbon having a cyclopentane skeleton has a smaller molecular volume than that of the fluorine-containing, cyclic, saturated hydrocarbon having cyclohexane as a skeleton for any substituent (R).

As described above, the cyclic, saturated hydrocarbon skeleton of the fluorine-containing, cyclic, saturated hydrocarbon of the present invention is a five-membered ring, and hence the hydrocarbon has a smaller molecular volume and smaller highest occupied molecular orbital energy than those of the fluorine-containing, cyclic, saturated hydrocarbon having a six-membered ring structure proposed in Patent Document No. 4 when the hydrocarbons have the same substituent (R).

A small molecular volume means that the molecular radius r is small, and as can be seen from the Einstein-Stokes equation represented by the formula (2), means that the diffusion property of a solvent molecule is high. In addition, the small molecular volume generally correlates with a small surface area of the molecule. When the surface area of the molecule reduces, an interaction occurring between the surface of the molecule and the surface of any other molecule reduces. In other words, when the surface area of the molecule reduces, the viscosity of a microscopic ambient environment where the solvent molecule is placed reduces. As can be seen from the formula (2), the diffusion property of the solvent molecule is improved as the viscosity $\eta$ of the microscopic ambient environment where the solvent molecule is placed reduces.

Those findings show that the fluorine-containing, cyclic, saturated hydrocarbon represented by the general formula (1) has high oxidation resistance, a high affinity for a polar solvent, and a low viscosity.

[Example 2]

2. Evaluation of fluorine-containing, cyclic, saturated hydrocarbon represented by general formula (1) for its compatibility with polar solvent

2.1 Preparation of sample

Synthesis of [trifluoromethylcyclopentane]

Trifluoromethylcyclopentane (TFMCP) was obtained by the following synthesis method.

3.4 Grams of cyclopentanecarboxylic acid (manufactured by KANTO CHEMICAL CO. , INC.) were housed in a 30-mL autoclave made of stainless steel with a valve, and were then cooled with a dry ice-acetone bath. The pressure in the autoclave was reduced, and then 10 g of $SF_4$ (manufactured by KANTO DENKA KOGYO CO., LTD.) were

introduced from the valve. The autoclave was hermetically sealed, and then the contents were caused to react with each other in a state heated to 130°C for 24 hours. After the reaction, a gas component was removed from the valve, and then the reaction mixture was washed with saturated baking soda water. The reaction mixture after the washing was purified with a spinning-band precision fractionating apparatus (manufactured by TAIKA). Thus, 2.4 g of a colorless liquid were obtained.

As a result of the measurement of a 1H-NMR spectrum ($CDCl_3$), a multiplet corresponding to eight hydrogen atoms having a peak at 1.50 to 1.92 ppm and a multiplet corresponding to one hydrogen atom having a peak at 2.46 to 2.66 ppm were observed, and hence the compound was found to have nine hydrogen atoms. In addition, as a result of the measurement of a 19F-NMR spectrum ($CDCl_3$), a doublet corresponding to three fluorine atoms having a peak at -71.78 ppm was observed.

The foregoing results showed that the colorless liquid was trifluoromethylcyclopentane. It should be noted that its purity measured by gas chromatography (involving using a gas chromatograph manufactured by Shimadzu Corporation) was 99.1%.

Synthesis of [1,1-bis(trifluoromethyl)cyclopentane]

1,1-Bis(trifluoromethyl)cyclopentane (11BTFMCP) was obtained by the following synthesis method.

4.7 Grams of cyclopentane-1,1-dicarboxylic acid (manufactured by Syntechem) were housed in a 30-mL autoclave made of stainless steel with a valve, and were then cooled with a dry ice-acetone bath. The pressure in the autoclave was reduced, and then 20 g of $SF_4$ (manufactured by KANTO DENKA KOGYO CO., LTD.) were introduced from the valve. The autoclave was hermetically sealed, and then the contents were caused to react with each other in a state heated to 120°C for 24 hours. After the reaction, a gas component was removed from the valve, and then the reaction mixture was washed with saturated baking soda water. The reaction mixture after the washing was purified with a spinning-band precision fractionating apparatus (manufactured by TAIKA). Thus, 3.5 g of a colorless liquid were obtained.

As a result of the measurement of a 1H-NMRspectrum ($CDCl_3$), a multiplet corresponding to four hydrogen atoms having a peak at 1.70 to 1.81 ppm and a multiplet corresponding to four hydrogen atoms having a peak at 2.00 to 2.10 ppm were observed, and hence the compound was found to have eight hydrogen atoms. In addition, as a result of the measurement of a 19F-NMR spectrum ($CDCl_3$), a singlet corresponding to six fluorine atoms having a peak at -72.79 ppm was observed.

The foregoing results showed that the colorless liquid was 1,1-bis(trifluoromethyl)cyclopentane. It should be noted that its purity measured by gas chromatography (involving using a gas chromatograph manufactured by Shimadzu Corporation) was 99.0%.

Synthesis of [2-fluoroethylcyclopentane]

2-Fluoroethylcyclopentane (2FECP) was obtained by the following synthesis method.

4.7 Grams of 2-cyclopentaneethanol (manufactured by Tokyo Chemical Industry Co., Ltd.) were housed in a 100-mL reaction vessel. 8.6 Milliliters of triethylamine (manufactured by Tokyo Chemical Industry Co., Ltd.) and 47 mL of dehydrated dichloromethane (manufactured by KANTO CHEMICAL CO., INC.) were added to the vessel, and then the solution was cooled to 5°C with ice. 3.8 Milliliters of methanesulfonyl chloride (manufactured by KANTO CHEMICAL CO., INC.) were slowly added to the solution, and then the mixture was stirred at 5°C for 1 hour. After the stirring, it was confirmed by silica gel thin-layer chromatography (TLC) with a mixed solvent containing hexane and ethyl acetate at a ratio of 3:1 as a developing solvent that the spot of a raw material disappeared and a new spot was formed. 50 Milliliters of distilled water were added to the solution to separate the solution into two layers, and then the organic layer was washed with a saturated salt solution. After the washing, anhydrous magnesium sulfate was added to the washed product, and then the mixture was dried under a high vacuum. Thus, 7.8 g of 2-cyclopentylethylmethane sulfonate showing a pale orange color were obtained. Its purity measured by gas chromatography (involving using a gas chromatograph manufactured by Shimadzu Corporation) was 97.0%.

7.8 Grams of 2-cyclopentylethylmethane sulfonate were housed in a 100-mL reaction vessel. 25.4 Grams of tetrabutylammonium fluoride (manufactured by Wako Pure Chemical Industries, Ltd.) and 12.0 g of acetonitrile were added to the vessel, and then the mixture was stirred at 50°C for 18 hours. It was confirmed by the gas chromatography analysis (involving using a gas chromatograph manufactured by Shimadzu Corporation) of the reaction mixture after the stirring that a peak derived from 2-cyclopentylethylmethane sulfonate disappeared and the reaction was completed. 20 Milliliters of distilled water and 30 mL of pentane (manufactured by KANTO CHEMICAL CO., INC.) were added to the reaction mixture to separate the mixture into two layers, and then the organic layer was washed with a saturated salt solution. After the washing, anhydrous magnesium sulfate was added to the washed product, and then the mixture was dried under a high vacuum. Further, pentane as a solvent was removed by distillation at a bath temperature of 60°C.

The remaining reaction mixture was purified with a spinning-band precision fractionating apparatus (manufactured by TAIKA). Thus, 2.8 g of a colorless liquid were obtained.

As a result of the measurement of a 1H-NMRspectrum ($CDCl_3$), six peaks obtained by coupling a triplet corresponding to two hydrogen atoms with a fluorine atom at 4.54, 4.52, 4.51, 4.42, 4.40, and 4.39 ppm, a multiplet corresponding to nine hydrogen atoms having a peak at 1.49 to 1.96 ppm, and a multiplet corresponding to two hydrogen atoms having a peak at 1.08 to 1.17 ppm were observed, and hence the compound was found to have thirteen hydrogen atoms. In addition, as a result of the measurement of a 19F-NMR spectrum ($CDCl_3$), a singlet corresponding to one fluorine atom having a peak at -218.4 ppm was observed.

The foregoing results showed that the colorless liquid was 2-fluoroethylcyclopentane. It should be noted that its purity measured by gas chromatography (involving using a gas chromatograph manufactured by Shimadzu Corporation) was 99.6%.

[Trifluoromethylcyclohexane]

A commercial product was used as trifluoromethylcyclohexane (TFMCH) (CAS RN: 401-75-2). The commercial product was purified with a spinning-band precision fractionating apparatus (manufactured by TAIKA). The purity of the resultant purified product measured with a gas chromatograph (manufactured by Shimadzu Corporation) was 99.5%.

[1,2-Bis(trifluoromethyl)cyclohexane]

Synthesis was performed by employing a synthesis method disclosed in the examples of Patent Document No. 4. It should be noted that the purity of the resultant measured with a gas chromatograph (manufactured by Shimadzu Corporation) was 99.0%.

[2-fluoroethylcyclohexane]

Synthesis was performed by employing a synthesis method disclosed in the examples of Patent Document No. 4. It should be noted that the purity of the resultant measured with a gas chromatograph (manufactured by Shimadzu Corporation) was 99.2%. 2.2 Evaluation for compatibility

The two fluorine-containing, cyclic, saturated hydrocarbons represented by the general formula (1) described above were each mixed with a compound to be generally used as a polar solvent, and then each fluorine-containing, cyclic, saturated hydrocarbon was evaluated for its compatibility.

<Compatibility of trifluoromethylcyclopentane>

Trifluoromethylcyclopentane (TFMCP) synthesized in the foregoing was mixed with propylene carbonate (PC) (commercial battery grade) at a mixing ratio (wt% ratio) of 5: 95, 10:90, 30:70, 50:50, 70:30, or 90:10, and then the states of the mixed bodies was observed. Table 2 shows the results. In Table 2, a mixed body that became transparent as a result of complete compatibilization was represented as "○," and a mixed body that became opaque owing to layer separation or incomplete compatibilization was represented as "×." Table 3, Table 4, and Table 5 in the following also show results by using the same symbols.

Table 2

| | Mixing ratio | | | | | |
|---|---|---|---|---|---|---|
| TFMCP | 5 | 10 | 30 | 50 | 70 | 90 |
| PC | 95 | 90 | 70 | 50 | 30 | 10 |
| Compatibility | ○ | ○ | ○ | ○ | ○ | ○ |

As can be seen from the results shown in Table 2, trifluoromethylcyclopentane and propylene carbonate were completely compatible with each other at all mixing ratios.

<Compatibility of 1,1-bis(trifluoromethyl)cyclopentane>

Mixed bodies of 1,1-bis(trifluoromethyl)cyclopentane (11BTFMCP) and propylene carbonate (PC) were similarly produced, and then the states of the mixed bodies were observed. Table 3 shows the results.

Table 3

| | Mixing ratio | | | | | |
|---|---|---|---|---|---|---|
| 11BTFMCP | 5 | 10 | 30 | 50 | 70 | 90 |
| PC | 95 | 90 | 70 | 50 | 30 | 10 |
| Compatibility | ○ | ○ | ○ | ○ | ○ | ○ |

As can be seen from the results shown in Table 3, 1,1-bis(trifluoromethyl)cyclopentane and propylene carbonate were completely compatible with each other at all mixing ratios.

<Compatibility of 2-fluoroethylcyclopentane>

Mixed bodies of 2-fluoxoethylcyclopentane (2FECP) and propylene carbonate (PC) were similarly produced, and then the states of the mixed bodies were observed. Table 4 shows the results.

Table 4

| | Mixing ratio | | | | | |
|---|---|---|---|---|---|---|
| 2FECP | 5 | 10 | 30 | 50 | 70 | 90 |
| PC | 95 | 90 | 70 | 50 | 30 | 10 |
| Compatibility | ○ | ○ | ○ | ○ | ○ | ○ |

As can be seen from the results shown in Table 4, 2-fluoroethylcyclopentane and propylene carbonate were completely compatible with each other at all mixing ratios.

An evaluation for compatibility between methylcyclopentane (MCP) free of any fluorine atom and propylene carbonate was performed as a comparative example. Table 5 shows the results. It should be noted that a commercial product (manufactured by KANTO CHEMICAL CO., INC.) purified with a spinning-band precision fractionating apparatus (manufactured by TAIKA) was used as methylcyclohexane. Its purity measured by gas chromatography (involving using a gas chromatograph manufactured by Shimadzu Corporation) was 99.1%.

Table 5

| | Mixing ratio | | | | | |
|---|---|---|---|---|---|---|
| MCP | 5 | 10 | 30 | 50 | 70 | 90 |
| PC | 95 | 90 | 70 | 50 | 30 | 10 |
| Compatibility | × | × | × | × | × | × |

As can be seen from the results shown in Table 5, methylcyclopentane and propylene carbonate were not compatible with each other at any mixing ratio.

Summarizing the foregoing results showed that the three fluorine-containing, cyclic, saturated hydrocarbons of this example were compatible with propylene carbonate at an arbitrary ratio. In addition, the summarization showed that methylcyclopentane was completely incompatible with propylene carbonate.

The dipole moment of 2-fluoroethylcyclopentane (2FECP) is 1.94 debye, which is the third smallest value among the fluorine-containing, cyclic, saturated hydrocarbons represented by the general formula (1) shown in Table 1. A polar solvent is used in the electrolytic solution of an electrical storage device, and compatibility with the polar solvent depends on the dipole moment of a solvent. The compatibility with the polar solvent may be higher as the dipole moment becomes larger. Accordingly, it is assumed from the results of Example 2 that at least a fluorine-containing, cyclic, saturated hydrocarbon represented by the general formula (1) having a dipole moment equal to or larger than the dipole moment of 2FECP, i.e., 1.94 debye shows good compatibility with a polar solvent to be generally used in the electrolytic solution of an electrical storage device such as PC.

In addition, as shown in Table 1, the dipole moment of (1-fluoroethyl) cyclohexane is 1.66 debye, and Example 1 of Patent Document No. 4 shows that (1-fluoroethyl)cyclohexane is compatible with PC except for the case where (1-fluoroethyl)cyclohexane is mixed with PC at a ratio of 50:50.

Meanwhile, a compound having the smallest dipole moment among the fluorine-containing, cyclic, saturated hydrocarbons represented by the general formula (1) shown in Table 1 is (1-fluoroethyl) cyclopentane, and its dipole moment is 1.66 debye.

Therefore, (1-fluoroethyl)cyclopentane may also show the same degree of compatibility with PC as that of (1-fluoroethyl)cyclohexane. In addition, any other fluorine-containing, cyclic, saturated hydrocarbon represented by the general formula (1) shown in Table 1 has a dipole moment of 1.66 debye or more. Accordingly, a fluorine-containing, cyclic, saturated hydrocarbon represented by the general formula (1) having a dipole moment of 1.66 debye or more and less than 1.94 debye except (1-fluoroethyl)cyclopentane may also show good compatibility with a polar solvent to be generally used in the electrolytic solution of an electrical storage device such as PC.

[Example 3]

3. Evaluations of fluorine-containing, cyclic, saturated hydrocarbon represented by general formula (1) for its viscosity and diffusion property

3.1 Evaluation for viscosity

TFMCP, 11BTFMCP, and 2FECP each used in Example 2 as the fluorine-containing, cyclic, saturated hydrocarbon of the present invention were evaluated for their viscosities. Also performed as comparative examples were the evaluations of trifluoromethylcyclohexane (TFMCH), 1,2-bis(trifluoromethyl)cyclohexane (12BTFMCH), and 2-fluoroethylcyclohexane (2FECH) identical in the kind and number of substituents to TFMCP, 11BTFMCP, and 2FECP, respectively for their viscosities.

A measurement was performed with a microchip type micro-sample viscometer (manufactured by RheoSense) in a room-temperature environment under the following conditions three times, and the average of the three measured values was defined as a viscosity. Table 6 shows the results.

| | |
|---|---|
| Chip type: | B |
| Syrine size: | 2.5 $cm^3$ |
| Flow channel depth: | 92 $\mu m$ |
| Flow rate: | 0.525 $cm^3/min$ |
| Shear rate: | 2,000 1/sec |

Table 6

| Substituent | | Structural formula | Compound Name | Viscosity [mPas] |
|---|---|---|---|---|
| One $CF_3$- | Example | 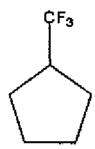 | Trifluoromethylcyclopentane | 0.69 |
| | Comparative example | 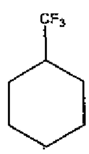 | Trifluoromethylcyclohexane | 0.97 |

(continued)

| Substituent | | Structural formula | Compound Name | Viscosity [mPas] |
|---|---|---|---|---|
| Two $CF_3$-'s | Example | 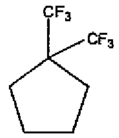 | 1,2-Bis(trifluoromethyl)cyc lopentane | 1.56 |
| | Comparative example | 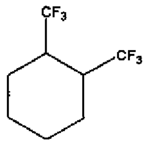 | 1,2-Bis(trifluoromethyl)cyc lohexane | 2.63 |
| One $CFH_2CH_2$- | Example | 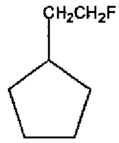 | 2-Fluoroethylcyclopentane | 0.84 |
| | Comparative example | 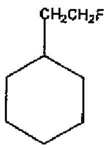 | 2-Fluoroethylcyclohexane | 1.31 |

As can be seen from the results of Table 6, when compounds identical in the kind and number of substituents to each other are compared with each other, the fluorine-containing, cyclic, saturated hydrocarbon having a cyclopentane ring (five-membered ring structure) of the present invention has a lower viscosity than that of a hydrocarbon having a cyclohexane ring (six-membered ring structure) as a comparative example. The fluorine-containing, cyclic, saturated hydrocarbon having a cyclopentane ring (five-membered ring structure) has a smaller molecular volume than that of a hydrocarbon having a cyclohexane ring (six-membered ring structure). Therefore, the surface area of a molecule reduces, and hence an interaction between the surface of a molecule and the surface of the other molecule occurring between adj acent molecules reduces. As a result, the viscosity may have reduced.

3.2 Evaluation for diffusion property

TFMCP, 11BTFMCP, and 2FECP each used in Example 2 as the fluorine-containing, cyclic, saturated hydrocarbon of the present invention were evaluated for their diffusion coefficients. Also performed as comparative examples were the evaluations of trifluoromethylcyclohexane (TFMCH), 1,2-bis(trifluoromethyl)cyclohexane (12BTFMCH), and 2-fluoroethylcyclohexane (2FECH) identical in the kind and number of substituents to TFMCP, 11BTFMCP, and 2FECP, respectively for their diffusion coefficients.

A measurement was performed by a pulsed-gradient spin-echo nuclear magnetic resonance method (PGSE-NMR). A nuclear magnetic resonance spectrometer manufactured by JEOL Ltd. was used, and the measurement was performed in an environment having a temperature of 30°Cwith 1H as an observation nucleus at an observation frequency of 395.8843 MHz. Detailed measurement conditions are described below. In addition, Table 7 shows the results of the evaluations.

| | |
|---|---|
| Pulse: | 90° pulse |
| Maximum magnetic field gradient intensity: | 0.8 T/m |
| Diffusion time: | 0.1 sec |
| Number of magnetic field gradient steps: | 16 steps |

Table 7

| Substituent | | Structural formula | Compound Name | Diffusion coefficient [$m^2s^{-1}$] |
|---|---|---|---|---|
| One $CF_3$- | Example | 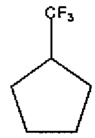 | Trifluoromethylcyclopentane | $18.3\times10^{-10}$ |
| | Comparative example | 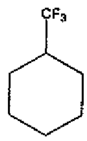 | Trifluoromethylcyclohexane | $13.6\times10^{-10}$ |
| Two $CF_3$-'s | Example | 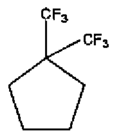 | 1,1-Bis(trifluoromethyl)cyclopen tane | $8.0\times10^{-10}$ |
| | Comparative example | 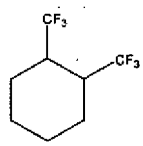 | 1,2-Bis(trifluoromethyl)cyclohex ane | 5.1x$_{IO}$-10 |
| One $CFH_2CH_2$- | Example | 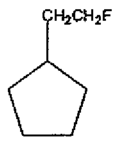 | 2-Fluoroethylcyclopentane | $15.0\times10^{-10}$ |
| | Comparative example | 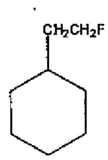 | 2-Fluoroethylcyclohexane | $10.0\times10^{-10}$ |

As can be seen from the results of Table 7, when compounds identical in the kind and number of substituents to each other are compared with each other, the fluorine-containing, cyclic, saturated hydrocarbon having a cyclopentane ring (five-membered ring structure) of this example has a larger diffusion coefficient than that of a hydrocarbon having a cyclohexane ring (six-membered ring structure) as a comparative example. The fluorine-containing, cyclic, saturated hydrocarbon having a cyclopentane ring (five-membered ring structure) has a smaller molecular volume than that of a hydrocarbon having a cyclohexane ring (six-membered ring structure). Therefore, the molecular radius reduces. It can be understood from the Einstein-Stokes equation represented by the formula (2) that causes for an improvement in the diffusion property of a fluorine-containing, cyclic, saturated hydrocarbon having a cyclopentane ring (five-membered ring structure) of this example are reductions in its molecular volume and viscosity as a result of the adoption of the pentane ring.

In addition, in consideration of the results of Example 1 (FIG. 2), when the fluorine-containing, cyclic, saturated hydrocarbon represented by the general formula (1) and the fluorine-containing, cyclic, saturated hydrocarbon having, as a skeleton, cyclohexane having the same substituent (R) are compared with each other, the fluorine-containing, cyclic, saturated hydrocarbon having a cyclopentane skeleton has a smaller molecular volume than that of the fluorine-containing, cyclic, saturated hydrocarbon having cyclohexane as a skeleton for any substituent (R). Accordingly, the former may have a smaller viscosity and a larger diffusion coefficient.

[Example 4]

4. Evaluation of solvent for electrical storage device for its oxidation resistance

A nonaqueous electrolytic solution was prepared with the solvent for an electrical storage device according to the present invention, and then the solvent for an electrical storage device was evaluated for its oxidation resistance by measuring a value for a current flowing upon application of a voltage to the nonaqueous electrolytic solution.

First, a triode glass cell 30 illustrated in FIG. 3 was prepared. The triode glass cell 30 has such a structure that a working electrode 36, a counter electrode 34 opposite to the working electrode 36, and a reference electrode 35 are placed in a glass container 38. A Pt plate measuring 1 cm by 1 cm (purity: 99.9 wt%) was used as the working electrode 36, an electrode obtained by crimping an Li foil 33b having a thickness of 150 μm onto a mesh 33a made of stainless steel (SUS304) measuring 2 cm by 2 cm was used as the counter electrode 34, and an Li wire having a diameter of 2 mm was used as the reference electrode 35. The working electrode 36 is connected to a Pt wire 37 and the counter electrode 34 is connected to a stainless wire 32. The Pt wire 37, the reference electrode 35, and the stainless wire 32 are fixed with a rubber plug 31.

Next, Example 4-1, Example 4-2, Example 4-3, Reference Examples 4-1 and 4-2, Comparative Example 4-1, and Comparative Example 4-2 were each prepared as a sample to be evaluated for its oxidation resistance.

(Example 4-1)

The electrolytic solution of Example 4-1 was prepared by dissolving $LiPF_6$ (commercial battery grade) as a supporting electrolyte in a mixed solvent containing TFMCP synthesized in Example 2 and diethyl carbonate (DEC) (commercial battery grade) at a volume ratio of 10:90. The concentration of $LiPF_6$ in the electrolytic solution was adjusted to 0.1 mol/L.

(Example 4-2)

The electrolytic solution of Example 4 - 2 was prepared by dissolving $LiPF_6$ (commercial battery grade) as a supporting electrolyte in a mixed solvent containing 11BTFMCP synthesized in Example 2 and diethyl carbonate (DEC) (commercial battery grade) at a volume ratio of 10:90. The concentration of $LiPF_6$ in the electrolytic solution was adjusted to 0.1 mol/L.

(Example 4-3)

The electrolytic solution of Example 4 - 3 was prepared by dissolving $LiPF_6$ (commercial battery grade) as a supporting electrolyte in a mixed solvent containing 2FECP synthesized in Example 2 and diethyl carbonate (DEC) (commercial battery grade) at a volume ratio of 10:90. The concentration of $LiPF_6$ in the electrolytic solution was adjusted to 0.1 mol/L.

(Reference Example 4-1)

The electrolytic solution of Reference Example 4-1 was prepared by dissolving $LiPF_6$ (commercial battery grade) as a supporting electrolyte in a mixed solvent containing TFMCH purified in Example 2 and diethyl carbonate (DEC) (commercial battery grade) at a volume ratio of 10:90. The concentration of $LiPF_6$ in the electrolytic solution was adjusted to 0.1 mol/L.

(Reference Example 4-2)

The electrolytic solution of Reference Example 4-2 was prepared by dissolving $LiPF_6$ (commercial battery grade) as a supporting electrolyte in a mixed solvent containing 12BTFMCH synthesized in Example 2 and diethyl carbonate (DEC) (commercial battery grade) at a volume ratio of 10:90. The concentration of $LiPF_6$ in the electrolytic solution was adjusted to 0.1 mol/L.

(Comparative Example 4-1)

The electrolytic solution of Comparative Example 4-1 was prepared by dissolving $LiPF_6$ (commercial battery grade) as a supporting electrolyte in a mixed solvent containing PC (commercial battery grade) and diethyl carbonate (DEC) (commercial battery grade) at a volume ratio of 10:90. The concentration of $LiPF_6$ in the electrolytic solution was

adjusted to 0.1 mol/L.

(Comparative Example 4-2)

The electrolytic solution of Comparative Example 4-2 was prepared by dissolving $LiPF_6$ (commercial battery grade) as a supporting electrolyte in a mixed solvent containing ethyl methyl carbonate (EMC) (commercial battery grade) and diethyl carbonate (DEC) (commercial battery grade) at a volume ratio of 10:90. The concentration of $LiPF_6$ in the electrolytic solution was adjusted to 0.1 mol/L.

As illustrated in FIG. 3, the electrolytic solution of each of Examples 4-1, 4-2, and 4-3, Reference Examples 4-1 and 4-2, and Comparative Examples 4-1 and 4-2 was injected into the triode glass cell 30 so that an evaluation cell was obtained. A voltage-current curve was measured with an electrochemical analyzer (manufactured by ALS) having a maximum bathvoltage of 26 V by a linear sweep voltammetry (LSV) method. The measurement was performed by sweeping the voltage of the working electrode with respect to the reference electrode from a natural open circuit voltage to 8 V at 5 mV/sec. It should be noted that a voltage-current curve showing the oxidation behavior of each of TFMCP, 11BTFMCP, 2FECP, TFMCH, 12BTFMCH, PC, and EMC was obtained by: separately preparing a blank electrolytic solution by dissolving 0.1 mol/L of $LiPF_6$ (commercial battery grade) as a supporting electrolyte in a DEC (commercial battery grade) single solvent; measuring its voltage-current curve by the LSV method; and subtracting the curve from the voltage-current curve of each of Examples 4-1, 4-2, and 4-3, Reference Examples 4-1 and 4-2, and Comparative Examples 4-1 and 4-2. FIG. 4 illustrates the results of those measurements. A current value measured by the LSV method is an indicator representing the rate of the oxidation reaction of a solvent, and hence a smaller current value means that the oxidation resistance of the solvent is more excellent.

As illustrated in FIG. 4, the current values of the electrolytic solutions of Examples 4-1, 4-2, and 4-3 increased little or reduced even when the voltage between the working electrode and the reference electrode increased. Increases in the current values of the electrolytic solutions of Examples 4-1, 4-2, and 4-3 are small as compared even with the increase behavior of the current value of the electrolytic solution of each of Reference Examples 4-1 and 4-2 each using a fluorine-containing, cyclic, saturated hydrocarbon having a cyclohexane ring (six-membered ring structure) disclosed to have high oxidation resistance in the examples of Patent DocumentNo. 4. In particular, the current values of the electrolytic solutions of Examples 4-2 and 4-3 showed negative values as the voltage between the working electrode and the reference electrode increased. This is because the current values of the evaluation cell using 11BTFMCP of Example 4-2 and the evaluation cell using 2FECP of Example 4-3 became smaller than the current value of the blank electrolytic solution containing only DEC as the voltage between the working electrode and the reference electrode increased. This means that the oxidation reaction of DEC is suppressed in each of the evaluation cells of Example 4-2 and Example 4-3, though the reason for the suppression is unclear.

In contrast, it is found that the currents in the electrolytic solutions of Comparative Examples 4-1 and 4-2 using cyclic and chain carbonates that have been conventionally used in a lithium ion secondary battery and an electric double layer capacitor start to increase at about 5.0 V.

Those results show that the electrolytic solutions of Examples 4-1, 4-2, and 4-3 each have extremely excellent oxidation resistance. In particular, it is found that the solvent of the present invention used in each of the examples has a small current value at a voltage between the working electrode and the reference electrode around 6 V that is of importance in a lithium ion secondary battery having a charging voltage of 4 to 5 V or an electric double layer capacitor to be charged to 2.5 V or more, and is hence excellent as a solvent for a high-voltage type electrical storage device.

In addition, as shown in Table 1, the highest occupied molecular orbital energy of 2FECP used in Example 4-3 is -8.26 eV, and hence 2FECP shows the second largest highest occupied molecular orbital energy among the fluorine-containing, cyclic, saturated hydrocarbons of the examples represented by the general formula (1). As described in the foregoing, the highest occupied molecular orbital energy and the oxidation resistance are related to each other, and a smaller highest occupied molecular orbital energy means that the oxidation resistance is more excellent. Therefore, at least a fluorine-containing, cyclic, saturated hydrocarbon of an example represented by the general formula (1) showing a highest occupied molecular orbital energy smaller than -8.26 eV may show excellent oxidation resistance. In addition, (1-fluoroethyl)cyclopentane shown in Table 1 is considered to show the same degree of oxidation resistance as that of 2FECP because its highest occupied molecular orbital energy, which is -8.25 eV, differs from that of 2FECP by only 0.01 eV.

[Example 5]

5. Evaluation for gas-generating ability

A lithium ion secondary battery was produced and charged at a high voltage. After that, the amount of a gas generated by immersing its charged positive electrode in the solvent for an electrical storage device according to the

present invention and holding the positive electrode at a high temperature was measured. A method of producing the lithium ion secondary battery used in the experiment and a procedure for the experiment are described with reference to FIG. 5 and FIG. 6. FIG. 5A is a schematic perspective view of the produced lithium ion secondary battery, and FIG. 5B and FIG. 5C each illustrate an I-I section in FIG. 5A. In addition, FIG. 5C illustrates the sectional structure of an electrode group. FIG. 6 is a flowchart illustrating the method of producing the lithium ion secondary battery and the procedure for the experiment.

<Production of positive electrode (step 101)>

First, $LiCoO_2$ (having an average particle diameter of 10 μm and a specific surface area according to a BET method of 0.38 $m^2/g$) was prepared as a positive electrode active material. 3 Parts by weight of acetylene black as a conductive agent, 4 parts by weight of polyvinylidene fluoride as a binder, and a proper amount of N-methyl-2-pyrrolidone were added to 100 parts by weight of the active material, and then the contents were stirred and mixed so that a slurry-like positive mix was obtained. It should be noted that polyvinylidene fluoride was used in a state of being dissolved in N-methyl-2-pyrrolidone in advance.

Next, as illustrated in FIG. 5C, the slurry-like positive mix was applied to one surface of a positive electrode collector 1a formed of an aluminum foil having a thickness of 20 μm, and then the applied film was dried and rolled with a roller. Thus, a positive electrode active material layer 1b was formed on the positive electrode collector 1a.

A method of preparing $LiCoO_2$ used as a positive electrode active material is as described below. An alkaline solution in which sodium hydroxide had been dissolved was dropped to a saturated aqueous solution of cobalt sulfate while the aqueous solution was stirred at a low speed. Thus, the precipitate of $Co(OH)_2$ was obtained. The precipitate was filtrated and washed with water, and was then dried by being heated to 80°C in the air. The resultant hydroxide had an average particle diameter of about 10 μm.

Next, the resultant hydroxide was subjected to a heat treatment at 380°C in the air for 10 hours. Thus, an oxide $Co_3O_4$ was obtained. Powder X-ray diffractometry confirmed that the resultant oxide had a single phase.

Further, the resultant oxide was mixed with a lithium carbonate powder so that a ratio between the number of moles of Co and the number of moles of Li was 1.00:1.00, and then the mixture was subjected to a heat treatment at 850 °C in dry air for 10 hours. Thus, target $LiCoO_2$ was obtained. A powder X-ray diffractometer (manufactured by Rigaku Corporation) confirmed that $LiCoO_2$ thus obtained had a single phase, hexagonal layer structure. $LiCoO_2$ was subjected to pulverization and classification treatments, and was then observed with a scanning electron microscope (manufactured by Hitachi High-Technologies Corporation). As a result, it was confirmed that $LiCoO_2$ had a particle diameter of about 6 to 15 μm. It should be noted that its average particle diameter was determined with a scanning particle size distribution-measuring apparatus (manufactured by HORIBA, Ltd.).

The resultant plate was punched into dimensions illustrated in FIG. 7A, and then the positive electrode active material layer 1b at a tab portion as a lead attachment portion was peeled. Thus, a positive electrode 1 was obtained. The positive electrode collector 1a provided with the positive electrode active material layer 1b has a rectangular shape measuring 30 mm by 40 mm.

<Production of negative electrode (step 102)>

First, a negative electrode collector 2a was formed by punching a mesh made of stainless steel (SUS304) into dimensions illustrated in FIG. 7B. The negative electrode collector 2a has an electrode portion having a rectangular shape measuring 31 mm by 41mm, and a lead attachment portion having a square shape measuring 7 mm by 7 mm. A negative electrode active material layer 2b formed of metal lithium having a thickness of 150 μm was crimped onto the electrode portion out of the negative electrode collector 2a. Thus, a negative electrode 2 was obtained.

<Assembly (step 103)>

As illustrated in FIG. 5C, the resultant positive electrode 1 and negative electrode 2 were laminated through a separator 3. Thus, the electrode group 13 was produced. A polyethylene microporous sheet having a thickness of 20 μm was used as the separator.

Next, as illustrated in FIG. 5A, a positive electrode lead 11 made of aluminum and a negative electrode lead 12 made of nickel were welded to the positive electrode 1 and negative electrode 2 of the electrode group 13, respectively. After that, the electrode group 13 was stored in a battery case 14 made of an aluminum laminate film having a thickness of 0.12 mm and opening in three directions, and was then fixed to the inner surface of the battery case 14 with a tape made of PP. Opening portions including the opening portion from which the positive electrode lead 11 and the negative electrode lead 12 were protruded were subjected to thermal welding, and only one opening portion was left without being subjected to thermal welding so that the battery case 14 was formed into a bag shape. As illustrated in FIG. 5B, a

predetermined amount of a nonaqueous electrolytic solution 15 was injected from the opening portion not subjected to thermal welding, and then decompression and deaeration were performed. After that, the opening portion was subjected to thermal welding in a decompressed state. Thus, the inside of the battery was hermetically sealed.

Used as the nonaqueous electrolytic solution 15 was a solution prepared by dissolving $LiPF_6$ (commercial battery grade) as a supporting electrolyte in a mixed solvent containing ethylene carbonate (commercial battery grade) (EC) and EMC (commercial battery grade) at a volume ratio of 1:3. $LiPF_6$ was dissolved so that its number of moles in the electrolytic solution was 1 mol/L.

<Charging (step 104)>

The battery produced in accordance with the steps 101 to 103 was charged to 4.4 V with a constant current at a current value of 4 mA. After that, the constant-voltage charged state at 4.4 V was kept until the current value attenuated to 0.8 mA.

<Decomposition (step 105)>

The battery after the completion of the charging was opened under an inert gas atmosphere having a dew point of -70 °C, and then the positive electrode 1 to which the positive electrode lead 11 had been welded was taken out. Next, the tab portion of the positive electrode 1 thus taken out was cut so that the positive electrode lead 11 was removed. Further, the positive electrode 1 from which the tab portion had been cut was immersed in dimethyl carbonate (DMC) (commercial battery grade) so that the electrolytic solution in the positive electrode 1 was extracted and removed. After that, the positive electrode 1 was taken out of DMC, and then DMC was removed by vacuum drying at room temperature. Thus, the positive electrode charged to a high voltage was obtained.

<High-temperature storage of solvent and charged positive electrode (step 106)>

Ten samples of Examples 5-1 to 5-6, Reference Examples 5-1 , 5-2, and Comparative Examples 5-1 and 5-2 were each produced by the following method as a sample to be subjected to an evaluation for a gas-generating ability at the time of high-temperature storage of the solvent in the presence of the charged positive electrode.

(Example 5-1)

The charged positive electrode was housed in a bag-shaped aluminum laminate film measuring 50 mm wide by 100 mm high and having one open side. After 3 mL of TFMCP synthesized in the foregoing had been injected as a solvent for an evaluation, the opening portion was subjected to thermal welding in a decompressed state. Thus, the aluminum laminate film was hermetically sealed.

(Example 5-2)

A mixture containing TFMCP synthesized in the foregoing and PC (commercial battery grade) at a weight ratio of 90:10 was used as a solvent for an evaluation. The other construction was the same as that of Example 5-1.

(Example 5-3)

A mixture containing TFMCP synthesized in the foregoing and PC (commercial battery grade) at a weight ratio of 50:50 was used as a solvent for an evaluation. The other construction was the same as that of Example 5-1.

(Example 5-4)

Admixture containing TFMCP synthesized in the foregoing and PC (commercial battery grade) at a weight ratio of 10:90 was used as a solvent for an evaluation. The other construction was the same as that of Example 5-1.

(Example 5-5)

A mixture containing TFMCP synthesized in the foregoing and PC (commercial battery grade) at a weight ratio of 5:95 was used as a solvent for an evaluation. The other construction was the same as that of Example 5-1.

(Example 5-6)

11BTFMCP synthesized in the foregoing was used as a solvent for an evaluation. The other construction was the same as that of Example 5-1.

(Example 5-7)

2FECP synthesized in the foregoing was used as a solvent for an evaluation. The other construction was the same as that of Example 5-1.

(Reference Example 5-1)

TFMCH synthesized in the foregoing was used as a solvent for an evaluation. The other construction was the same as that of Example 5-1.

(Reference Example 5-2)

12BTFMCH synthesized in the foregoing was used as a solvent for an evaluation. The other construction was the same as that of Example 5-1.

(Reference Example 5-3)

2FECH synthesized in the foregoing was used as a solvent for an evaluation. The other construction was the same as that of Example 5-1.

(Comparative Example 5-1)

PC (commercial battery grade) was used as a solvent for an evaluation. The other construction was the same as that of Example 5-1.

(Comparative Example 5-2)

EMC (commercial battery grade) was used as a solvent for an evaluation. The other construction was the same as that of Example 5-1.

The twelve samples of Examples 5-1 to 5-7, Reference Examples 5-1 to 5-3, and Comparative Examples 5-1 and 5-2, i.e., the hermetically sealed aluminum laminate films were each placed in a thermostat and held at 85°C for 3 days. After that, the samples were each taken out of the thermostat, and then the quantitative analysis of a generated gas was performed with a gas chromatograph (manufactured by Shimadzu Corporation). Table 8 shows the total amount of the generated gas calculated from the result.

Table 8

| | Storage voltage [V] | Kind of solvent | Mixing ratio | Total amount of generated gas [$cm^3$] |
|---|---|---|---|---|
| Example 5-1 | 4.4 | TFMCP | 100 | 0.03 |
| Example 5-2 | | TFMCP:PC | 90:10 | 0.15 |
| Example 5-3 | | TFMCP:PC | 50:50 | 0.62 |
| Example 5-4 | | TFMCP:PC | 10:90 | 1.07 |
| Example 5-5 | | TFMCP:PC | 5:95 | 1.14 |
| Example 5-6 | | 11BTFMCP | 100 | 0.02 |
| Example 5-7 | | 2FECP | 100 | 0.02 |
| Reference Example 5-1 | | TFMCH | 100 | 0.05 |
| Reference Example 5-2 | | 12BTFMCH | 100 | 0.02 |
| Reference Example 5-3 | | 2FECH | 100 | 0.03 |
| Comparative Example 5-1 | | PC | 100 | 1.22 |
| Comparative Example 5-2 | | EMC | 100 | 1.37 |

As shown in Table 8, the amount of the generated gas is extremely small in each of Example 5-1 containing onlyTFMCPas a solvent, Example 5-6 containing only 11BTFMCP as a solvent, and Example 5-7 containing only 2FECP as a solvent, TFMCP, 11BTFMCP, and 2FECP serving as the fluorine-containing, cyclic, saturated hydrocarbons represented by the general formula (1). The amounts of the gases generated in those examples are identical to those of TFMCH, 12BTFMCH, and 2FECH shown in Reference Examples 5-1, 5-2, and 5-3, respectively disclosed by Patent Document No. 4 to generate no gases. Therefore, it is found that the fluorine-containing, cyclic, saturated hydrocarbon having a cyclopentane ring (five-membered ring structure) of the present invention represented by the general formula (1) also does not generate any gas. In other words, the hydrocarbon has so high oxidation resistance that the solvent does not decompose and hence a gas as a decomposition product may not be generated.

In consideration of the values for their highest occupied molecular orbital energies calculated in Example 1, none of TFMCP 11BTFMCP, and 2FECP is theoretically oxidized at a charging voltage of 4.4 V. In addition, even when the hydrocarbons are oxidized, the hydrocarbons do not generate $CO_2$ because the hydrocarbons are free of any carbonate structure in their molecular structures. Accordingly, it is probably because the electrolytic solution (containing a carbonate) used upon production of the charged positive electrode remains and is decomposed that a trace amount, specifically 0.02 to 0.03 $cm^3$ of the gas is generated in each of Examples 5-1, 5-6, and 5-7.

In each of Examples 5-2 to 5-5, the amount of the generated gas also increases as the content of PC in the solvent of the electrolytic solution increases. In consideration of the results of Examples 5-1 and 5-6, this is probably because the amount of the generated gas is derived from added PC and PC is decomposed by oxidation. In any one of the examples, however, the generation amount is smaller than those of Comparative Examples 5-1 and 5-2 using cyclic and chain carbonates that have been conventionally used in a lithium ion secondary battery and an electric double layer capacitor. Those results show that as long as 5 wt% or more of TFMCP is incorporated, the generation amount of the gas is reduced to 1.14 $cm^3$ or less (the generation amount is reduced by 5% or more) and hence a significant oxidation resistance effect is obtained. The results also show that as long as 10 wt% or more of TFMCP is incorporated, the generation amount of the gas is reduced to 1.07 $cm^3$ or less (the generation amount is reduced by 10% or more) and hence a remarkable oxidation resistance effect is obtained. In other words, it is found that the content of TFMCP in the solvent is preferably 5% or more, more preferably 10% or more.

In addition, it is assumed from the values for the highest occupied molecular orbital energies shown in Table 1 and the results of Example 4 that fluorine-containing, cyclic, saturated hydrocarbons represented by the general formula (1) except TFMCP, 11BTFMCP, and 2FECP are also not oxidized at a charging voltage of 4.4 V and hence do not generate gases as decomposition products. In order that a sufficient oxidation resistance effect may be obtained, any such fluorine-containing, cyclic, saturated hydrocarbon may also be incorporated into the solvent at preferably 5 wt% or more, more preferably 10 wt% or more.

As described in the foregoing, it has been revealed that the fluorine-containing, cyclic, saturated hydrocarbons represented by the general formula (1) including TFMCP, 11BTFMCP, 2FECP, and other hydrocarbons each have higher oxidation resistance than that of each of a cyclic carbonate typified by PC and a chain carbonate typified by EMC, and each have such excellent reliability that no gas is generated even when a high voltage is applied.

## INDUSTRIAL APPLICABILITY

The nonaqueous solvent for an electrical storage device of the present invention is useful as the solvent of an electrolytic solution for embodying an ultrahigh-voltage type nonaqueous electrical storage device having a high energy density. The solvent can also be used as a solvent for a conventional-voltage type lithium ion secondary battery or electric double layer capacitor, and realizes high high-temperature reliability. In particular, the solvent is suitable as the solvent of an electrolytic solution for a large battery or electric vehicle battery requested to have high reliability.

## REFERENCE SIGNS LIST

| | |
|---|---|
| 1 | positive electrode |
| 1a | positive electrode collector |
| 1b | positive electrode active material layer |
| 2 | negative electrode |
| 2a | negative electrode collector |
| 2b | negative electrode active material layer |
| 3 | separator |
| 11 | positive electrode lead |
| 12 | negative electrode lead |
| 13 | electrode group |
| 14 | battery case |
| 15 | nonaqueous electrolytic solution |
| 30 | triode glass cell |
| 31 | rubber plug |
| 32 | stainless wire |
| 33a | mesh made of stainless steel |
| 33b | Li foil |
| 34 | counter electrode |
| 35 | reference electrode |
| 36 | working electrode |
| 37 | Pt wire |
| 38 | glass container |

## Claims

1. Use of a nonaqueous solvent, comprising a fluorine-containing, cyclic, saturated hydrocarbon represented by the following general formula (1) and having a structure obtained by introducing one or two substituents R into a cyclopentane ring:

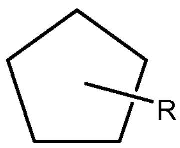

··· (1)

in the general formula (1), R is represented by $C_nX_{2n+1}$, n represents 1 or 2, at least one of the 2n+1 X's represents F, and the other X's each represent H, for an electrical storage device.

2. Use of a nonaqueous solvent for an electrical storage device according to claim 1, wherein the fluorine-containing, cyclic, saturated hydrocarbon comprises trifluoromethylcyclopentane.

3. Use of a nonaqueous solvent for an electrical storage device according to claim 1, wherein the fluorine-containing, cyclic, saturated hydrocarbon comprises 1,1-bis(trifluoromethyl)cyclopentane.

4. Use of a nonaqueous solvent for an electrical storage device according to claim 1, wherein the fluorine-containing,

cyclic, saturated hydrocarbon comprises 2-fluoroethylcyclopentane.

**5.** Use of a nonaqueous solvent for an electrical storage device according to one of claims 1 to 3, wherein the solvent contains 5 wt% or more and 100 wt% or less of the fluorine-containing, cyclic, saturated hydrocarbon as a solvent component.

**6.** Use of a nonaqueous solvent for an electrical storage device according to one of claims 1 to 3, wherein the solvent contains 10 wt% or more and 100 wt% or less of the fluorine-containing, cyclic, saturated hydrocarbon as a solvent component.

**7.** A nonaqueous electrolytic solution of an electrical storage device, comprising:

a supporting electrolyte;
at least one of a cyclic carbonate and a chain carbonate; and
a fluorine-containing, cyclic, saturated hydrocarbon represented by the following general formula (1) and having a structure obtained by introducing one or two substituents R into a cyclopentane ring:

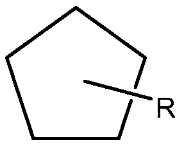 ··· (1)

in the general formula (1), R is represented by $C_nX_{2n+1}$, n represents 1 or 2, at least one of the 2n+1 X's represents F, and the other X's each represent H.

**8.** A nonaqueous electrolytic solution of an electrical storage device according to claim 7, wherein the fluorine-containing, cyclic, saturated hydrocarbon comprises trifluoromethylcyclopentane.

**9.** A nonaqueous electrolytic solution of an electrical storage device according to claim 7, wherein the fluorine-containing, cyclic, saturated hydrocarbon comprises 1,1-bis(trifluoromethyl)cyclopentane.

**10.** A nonaqueous electrolytic solution of an electrical storage device according to claim 1, wherein the fluorine-containing, cyclic, saturated hydrocarbon comprises 2-fluoroethylcyclopentane.

**11.** A nonaqueous electrolytic solution of an electrical storage device according to one of claims 7 to 9, wherein the nonaqueous electrolytic solution contains 5 wt% or more and 100 wt% or less of the fluorine-containing, cyclic, saturated hydrocarbon.

**12.** A nonaqueous electrolytic solution of an electrical storage device according to one of claims 7 to 9, wherein the nonaqueous electrolytic solution contains 10 wt% or more and 100 wt% or less of the fluorine-containing, cyclic, saturated hydrocarbon.

## Patentansprüche

**1.** Verwendung eines nichtwässrigen Lösungsmittels für eine elektrische Speichervorrichtung, umfassend
einen fluorhaltigen, zyklischen, gesättigten Kohlenwasserstoff dargestellt durch die folgende allgemeine Formel (1) und mit einer Struktur erhalten durch Einführen eines oder zwei Substituenten R in einen Cyclopentanring:

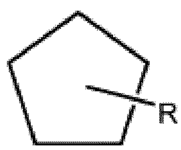 ··· (1)

in der allgemeinen Formel (1), ist R dargestellt durch $C_nX_{2n+1}$, stellt n 1 oder 2 dar, zumindest einer von den 2n+1 X'en stellt F dar und die anderen X'en stellen jeweils H dar.

2. Verwendung eines nichtwässrigen Lösungsmittels für eine elektrische Speichervorrichtung nach Anspruch 1, wobei der fluorhaltige, zyklische, gesättigte Kohlenwasserstoff Trifluoromethylcyclopentan umfasst.

3. Verwendung eines nichtwässrigen Lösungsmittels für eine elektrische Speichervorrichtung nach Anspruch 1, wobei der fluorhaltige, zyklische, gesättigte Kohlenwasserstoff 1,1-Bis(trifluoromethyl)cyclopentan umfasst.

4. Verwendung eines nichtwässrigen Lösungsmittels für eine elektrische Speichervorrichtung nach Anspruch 1, wobei der fluorhaltige, zyklische, gesättigte Kohlenwasserstoff 2-Fluoroethylcyclopentan umfasst.

5. Verwendung eines nichtwässrigen Lösungsmittels für eine elektrische Speichervorrichtung nach einem der Ansprüche 1 bis 3, wobei das Lösungsmittel 5 Gew.-% oder mehr und 100 Gew.-% oder weniger des fluorhaltigen, zyklischen, gesättigten Kohlenwasserstoffs als eine Lösungsmittelkomponente enthält.

6. Verwendung eines nichtwässrigen Lösungsmittels für eine elektrische Speichervorrichtung nach einem der Ansprüche 1 bis 3, wobei das Lösungsmittel 10 Gew.-% oder mehr und 100 Gew.-% oder weniger des fluorhaltigen, zyklischen, gesättigten Kohlenwasserstoffs als eine Lösungsmittelkomponente enthält.

7. Nichtwässrige Elektrolytlösung einer elektrischen Speichervorrichtung, umfassend:

einen Trägerelektrolyten;
zumindest eines von einem zyklischen Carbonat und einem Kettencarbonat; und
einen fluorhaltigen, zyklischen, gesättigten Kohlenwasserstoff, dargestellt durch die folgende allgemeine Formel (1) und mit einer Struktur erhalten durch Einführen eines oder zwei Substituenten R in einen Cyclopentanring :

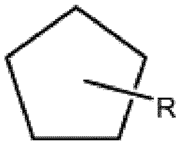 ··· (1)

in der allgemeinen Formel (1), ist R durch $C_nX_{2n+1}$ dargestellt, stellt n 1 oder 2 dar, und zumindest einer von den 2n+1 X'en stellt F dar, und die anderen X'en stellen jeweils H dar.

8. Nichtwässrige Elektrolytlösung einer elektrischen Speichervorrichtung nach Anspruch 7, wobei der fluorhaltige, zyklische, gesättigte Kohlenwasserstoff Trifluoromethylcyclopentan umfasst.

9. Nichtwässrige Elektrolytlösung einer elektrischen Speichervorrichtung nach Anspruch 7, wobei der fluorhaltige, zyklische, gesättigte Kohlenwasserstoff 1,1-Bis(trifluoromethyl)cyclopentan umfasst.

10. Nichtwässrige Elektrolytlösung einer elektrischen Speichervorrichtung nach Anspruch 1, wobei der fluorhaltige, zyklische, gesättigte Kohlenwasserstoff 2-Fluoroethylcyclopentan umfasst.

11. Nichtwässrige Elektrolytlösung einer elektrischen Speichervorrichtung nach einem der Ansprüche 7 bis 9, wobei die nichtwässrige Elektrolytlösung 5 Gew.-% oder mehr und 100 Gew.-% oder weniger des fluorhaltigen, zyklischen, gesättigten Kohlenwasserstoffs enthält.

12. Nichtwässrige Elektrolytlösung einer elektrischen Speichervorrichtung nach einem der Ansprüche 7 bis 9, wobei die nichtwässrige Elektrolytlösung 10 Gew.-% oder mehr und 100 Gew.-% oder weniger des fluorhaltigen, zyklischen, gesättigten Kohlenwasserstoffs enthält.

## Revendications

1. Utilisation d'un solvant non aqueux, comprenant un hydrocarbure saturé, cyclique, contenant du fluor, représenté par la formule générale (1) suivante et ayant une structure obtenue en introduisant un ou deux substituants R dans un cycle cyclopentane :

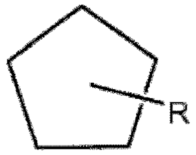

(1)

dans la formule générale (1), R est représenté par $C_nX_{2n+1}$, n représente 1 ou 2, au moins l'un des 2n+1 X représente un F, et les autres X représentent chacun un H, pour un dispositif de stockage électrique.

2. Utilisation d'un solvant non aqueux pour un dispositif de stockage électrique selon la revendication 1, dans lequel l'hydrocarbure saturé, cyclique, contenant du fluor comprend du trifluorométhylcyclopentane.

3. Utilisation d'un solvant non aqueux pour un dispositif de stockage électrique selon la revendication 1, dans laquelle l'hydrocarbure saturé, cyclique, contenant du fluor comprend du 1,1-bis(trifluorométhyl)cyclopentane.

4. Utilisation d'un solvant non aqueux pour un dispositif de stockage électrique selon la revendication 1, dans laquelle l'hydrocarbure saturé, cyclique, contenant du fluor comprend du 2-fluoroéthylcyclopentane.

5. Utilisation d'un solvant non aqueux pour un dispositif de stockage électrique selon l'une des revendications 1 à 3, dans laquelle le solvant contient 5 % en poids ou plus et 100 % en poids ou moins de l'hydrocarbure saturé, cyclique, contenant du fluor en tant que constituant solvant.

6. Utilisation d'un solvant non aqueux pour un dispositif de stockage électrique selon l'une des revendications 1 à 3, dans laquelle le solvant contient 10 % en poids ou plus et 100 % en poids ou moins de l'hydrocarbure saturé, cyclique, contenant du fluor en tant que constituant solvant.

7. Solution électrolytique non aqueuse d'un dispositif de stockage électrique, comprenant :

un électrolyte de support ;
au moins l'un d'un carbonate cyclique et d'un carbonate à chaîne ; et
un hydrocarbure saturé, cyclique, contenant du fluor représenté par la formule générale (1) suivante et ayant une structure obtenue en introduisant un ou deux substituants R dans un cycle cyclopentane :

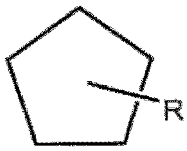

(1)

dans la formule générale (1), R est représenté par $C_nX_{2n+1}$, n représente 1 ou 2, au moins l'un des 2n+1 X représente un F, et les autres X représentent chacun un H.

8. Solution électrolytique non aqueuse d'un dispositif de stockage électrique selon la revendication 7, dans laquelle l'hydrocarbure saturé, cyclique, contenant du fluor comprend du trifluorométhylcyclopentane.

9. Solution électrolytique non aqueuse d'un dispositif de stockage électrique selon la revendication 7, dans laquelle l'hydrocarbure saturé, cyclique, contenant du fluor comprend du 1,1-bis(trifluorométhyl)cyclopentane.

10. Solution électrolytique non aqueuse d'un dispositif de stockage électrique selon la revendication 1, dans laquelle l'hydrocarbure saturé, cyclique, contenant du fluor comprend du 2-fluoroéthylcyclopentane.

11. Solution électrolytique non aqueuse d'un dispositif de stockage électrique selon l'une des revendications 7 à 9, dans laquelle la solution électrolytique non aqueuse contient 5 % en poids ou plus et 100 % en poids ou moins de l'hydrocarbure saturé, cyclique, contenant du fluor.

12. Solution électrolytique non aqueuse d'un dispositif de stockage électrique selon l'une des revendications 7 à 9, dans laquelle la solution électrolytique non aqueuse contient 10 % en poids ou plus et 100 % en poids ou moins de l'hydrocarbure saturé, cyclique, contenant du fluor.

*FIG.1*

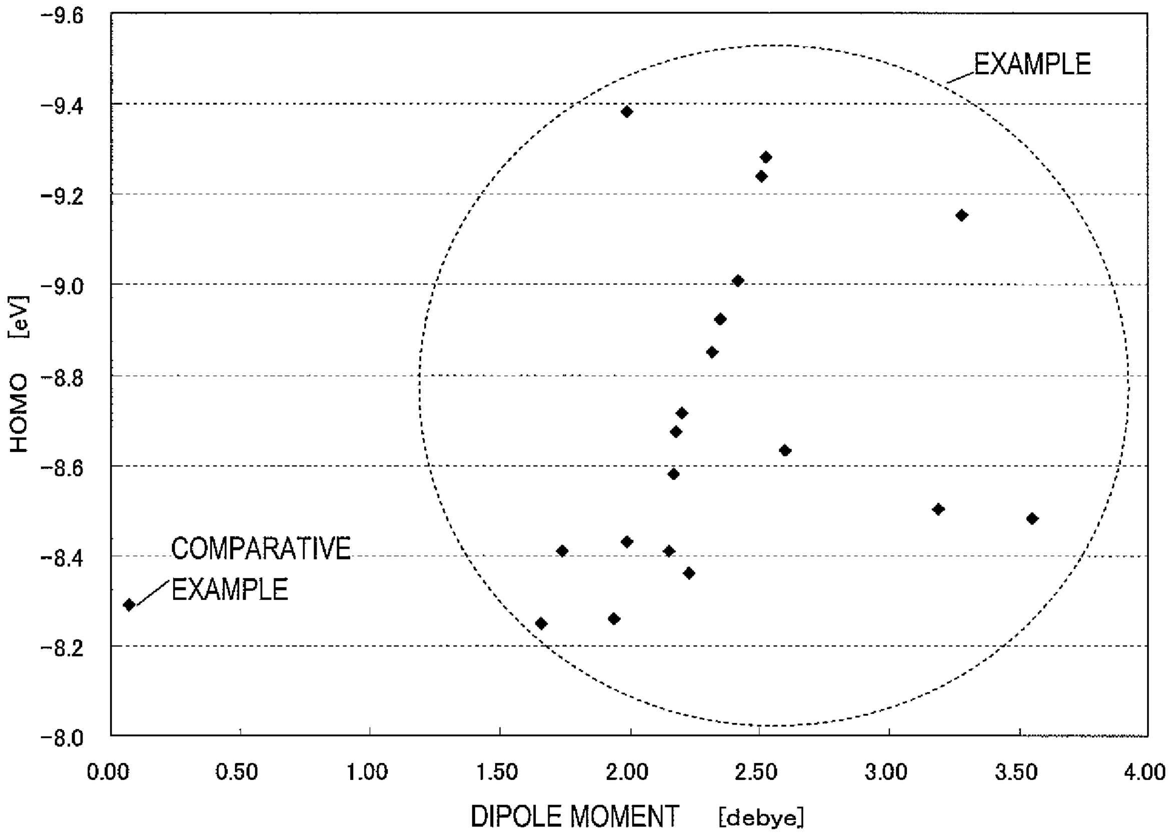
EXAMPLE
COMPARATIVE
EXAMPLE
HOMO [eV]
-9.6
-9.4
-9.2
-9.0
-8.8
-8.6
-8.4
-8.2
-8.0
0.00
0.50
1.00
1.50
2.00
2.50
3.00
3.50
4.00
DIPOLE MOMENT [debye]

*FIG.2*

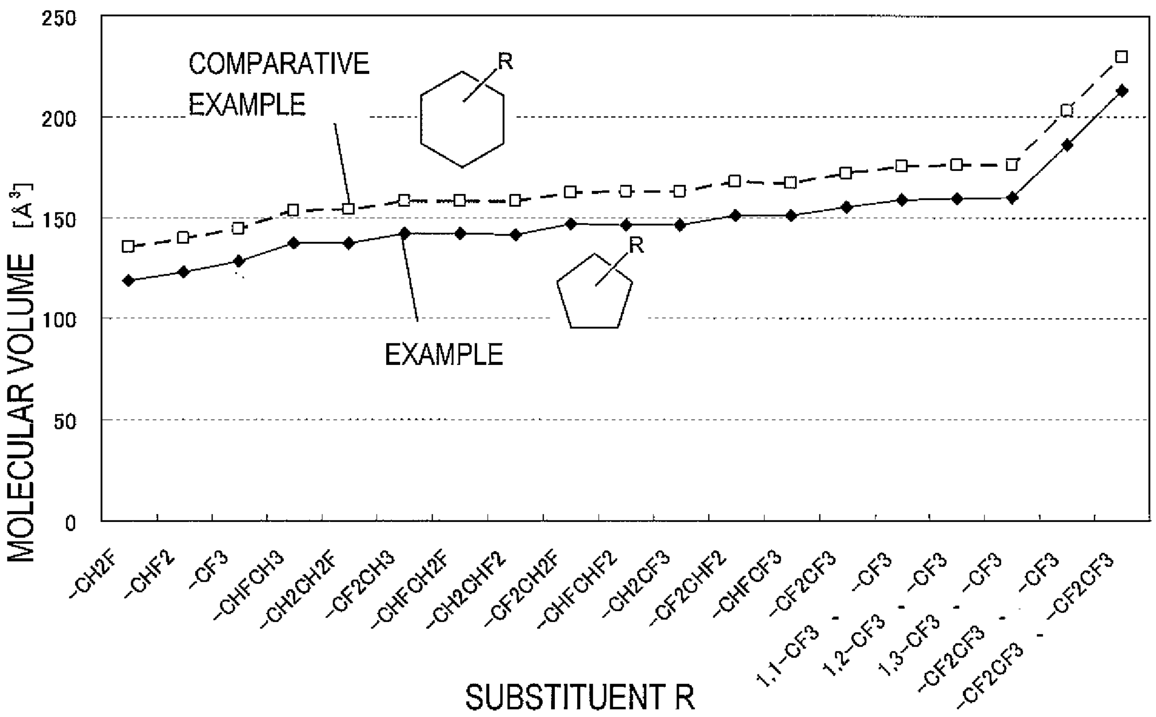
COMPARATIVE
EXAMPLE
R
R
EXAMPLE
MOLECULAR VOLUME [Å³]
250
200
150
100
50
0
-CH2F
-CHF2
-CF3
-CHFCH3
-CH2CH2F
-CF2CH3
-CHFCH2F
-CH2CHF2
-CF2CH2F
-CHFCHF2
-CH2CF3
-CF2CHF2
-CHFCF3
-CF2CF3
1,1-CF3 - -CF3
1,2-CF3 - -CF3
1,3-CF3 - -CF3
-CF2CF3 - -CF3
-CF2CF3 - -CF2CF3
SUBSTITUENT R

*FIG.3*

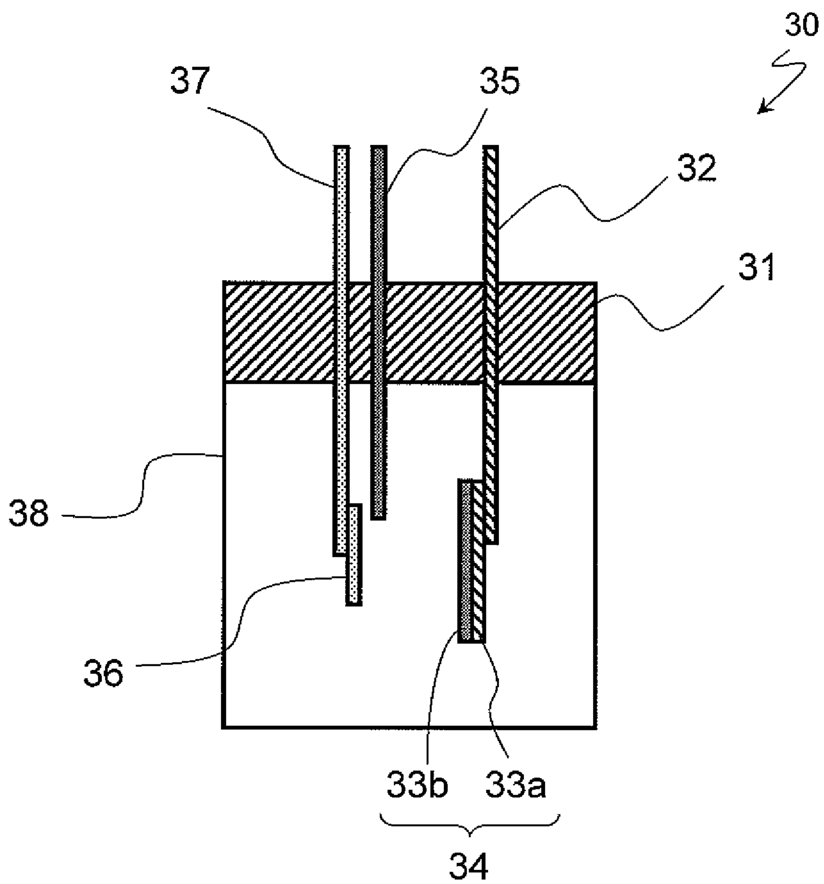

*FIG.4*

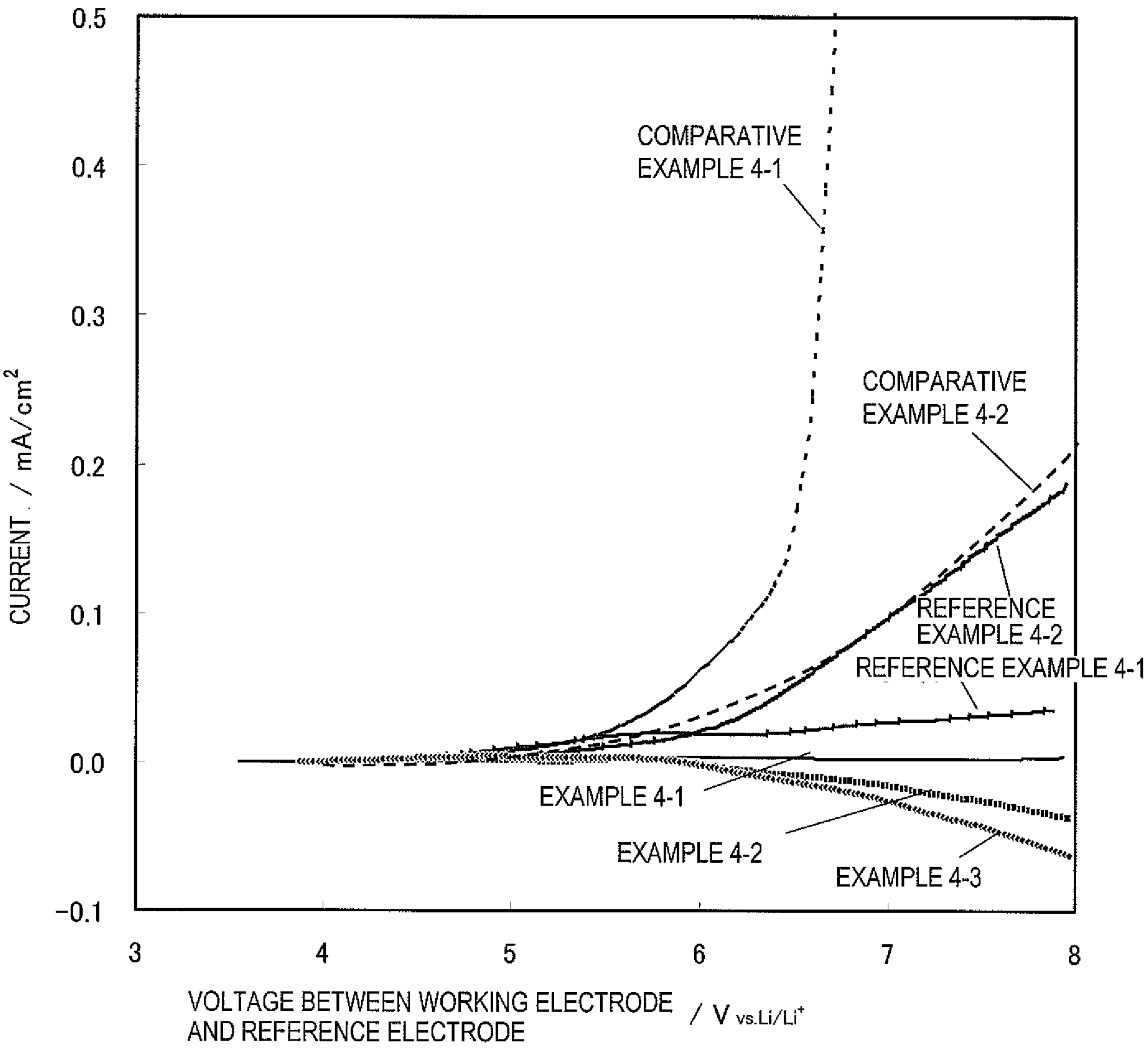
0.5
0.4
0.3
0.2
0.1
0.0
−0.1
CURRENT / mA/cm2
COMPARATIVE EXAMPLE 4-1
COMPARATIVE EXAMPLE 4-2
REFERENCE EXAMPLE 4-2
REFERENCE EXAMPLE 4-1
EXAMPLE 4-1
EXAMPLE 4-2
EXAMPLE 4-3
3
4
5
6
7
8
VOLTAGE BETWEEN WORKING ELECTRODE AND REFERENCE ELECTRODE / V vs.Li/Li+

*FIG.5*

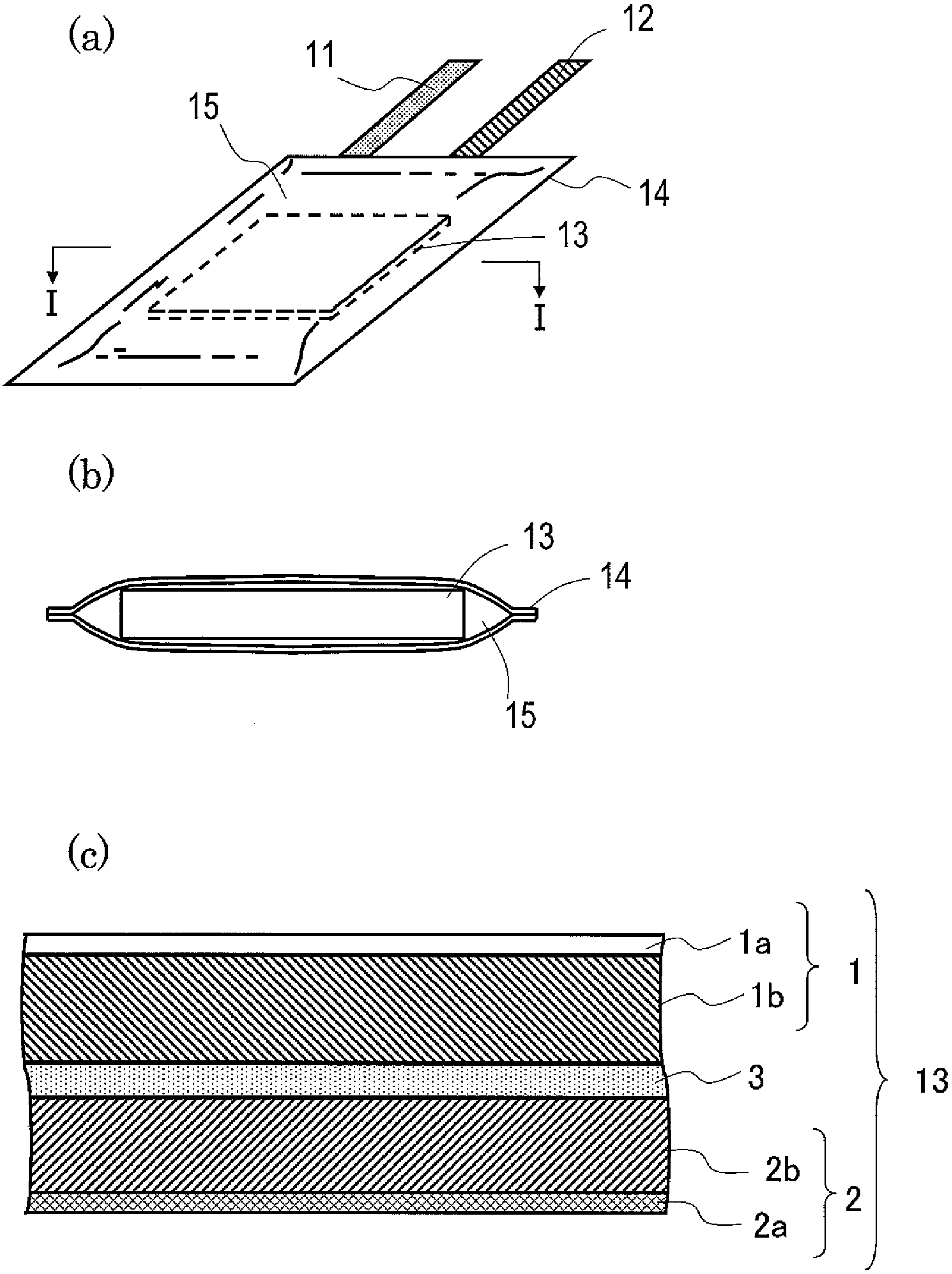

FIG.6

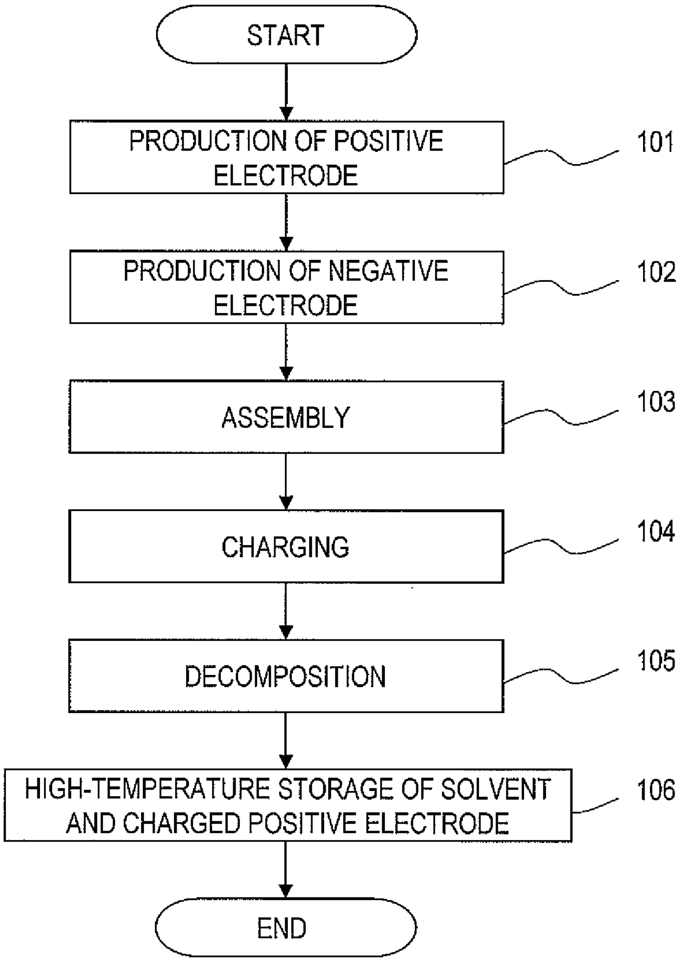
START
PRODUCTION OF POSITIVE ELECTRODE
101
PRODUCTION OF NEGATIVE ELECTRODE
102
ASSEMBLY
103
CHARGING
104
DECOMPOSITION
105
HIGH-TEMPERATURE STORAGE OF SOLVENT AND CHARGED POSITIVE ELECTRODE
106
END

FIG.7

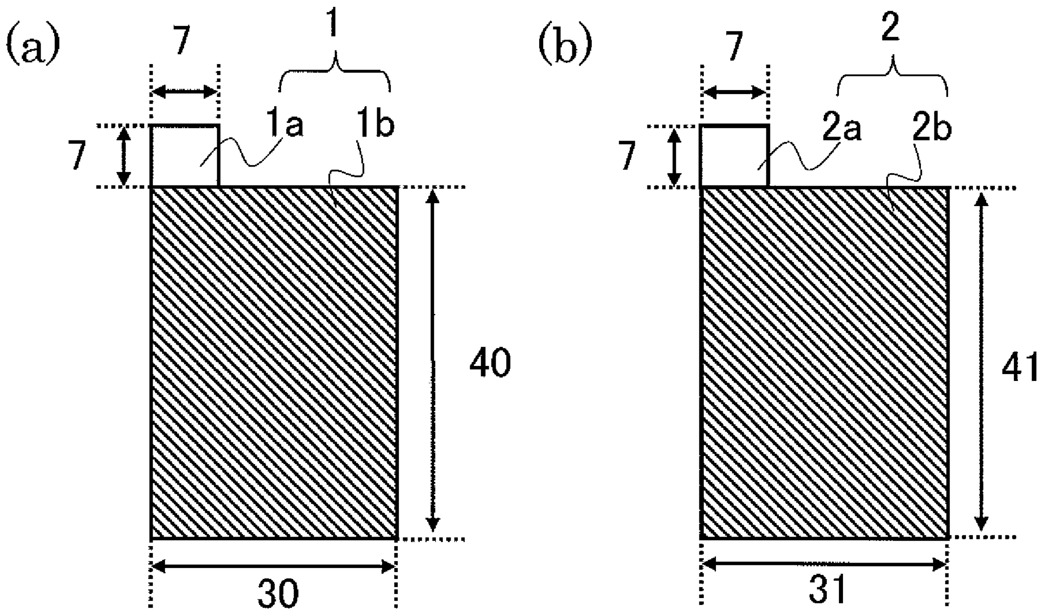
(a)
7
1
7
1a
1b
40
30
(b)
7
2
7
2a
2b
41
31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1557404 A1
- EP 1560236 A1
- JP 2001143749 A
- JP 2000348762 A
- JP 2000223151 A
- JP 2006172775 A
- JP 2006092815 A
- JP 2005149750 A
- JP 2004111359 A
- JP 2006286650 A
- WO 2009141999 A
- US 20090297954 A


### Non-patent literature cited in the description


- **M.B. EWING et al.** *THE JOURNAL OF CHEMICAL THERMODYNAMICS,* 01 February 1998, vol. 30 (2), 189-198
- **W. DMOWSKI et al.** *JOURNAL OF FLUORINE CHEMISTRY,* 01 March 2000, vol. 102 (1-2), 141-146
- **C. MELERO et al.** *CHEMISTRY - A EUROPEAN JOURNAL,* 06 November 2007, vol. 13 (36), 10096-10107
- **T.B. PATRICK et al.** *THE JOURNAL OF ORGANIC CHEMISTRY,* 01 January 1993, vol. 58 (3), 705-708
- **J. GONZALEZ et al.** *THE JOURNAL OF ORGANIC CHEMISTRY,* 01 June 1991, vol. 56 (13), 4322-4325